# EUROPEAN PATENT APPLICATION

(11) **EP 3 029 067 A1**
(43) Date of publication of application: **08.06.2016**
(21) Application number: 14195645.8
(22) Date of filing: 01.12.2014
(51) Int. Cl.: C07K 16/24, A61K 39/395

(54) **Use of blocking-reagents for reducing unspecific T cell-activation**

(71) Applicant: Deutsches Krebsforschungszentrum, 69120 Heidelberg (DE); Eberhard Karls Universität Tübingen, 72074 Tübingen (DE)
(72) Inventor: Jung, Gundram, 72108 Rottenburg-Wendelsheim (DE); Salih, Helmut, 72072 Tübingen (DE); Vogt, Fabian, 72072 Tübingen (DE); Kauer, Joseph, 72076 Tübingen (DE)
(74) Representative: Schiweck, Weinzierl & Koch

(57) **Abstract**

The present invention relates to a blocking-reagent for use in reducing unspecific T cell activation in T cell engaging therapies. The present invention further relates to pharmaceutical kit of parts and an *in vitro* method for evaluating unspecific T cell activation.

## Description

### FIELD OF THE INVENTION

The present invention relates to blocking-reagents for use in reducing unspecific T cell activation in T cell engaging therapies. The present invention further relates to pharmaceutical kit of parts and an *in vitro* method for evaluating unspecific T cell activation.

### BACKGROUND

Scientific work starting in the mid-eighties has established that bispecific antibody molecules directed to a target cell associated antigens, such as a tumor associated antigen (TAA), and the T cell receptor (TCR)/CD3-complex are capable of activating T cells and mediate the lysis of e.g. the tumor associated antigen (TAA) expressing tumor cells by the activated T cells (effector cells) (Staerz UD, Kanagawa O, Bevan MJ. Hybrid antibodies can target sites for attack by T cells. Nature 1985; 314:628-631; Perez P, Hoffman RW, Shaw S, Bluestone JA, Segal DM. Specific targeting of cytotoxic T cells by anti-T3 linked to anti-target cell antibody. Nature 1985; 316:354-356; Jung G, Honsik CJ, Reisfeld RA and Müller-Eberhard HJ. Activation of human peripheral blood mononuclear cells by anti-T3: Killing of tumor target cells coated with anti-target X anti-T3-conjugates. Proc Natl Acad Sci USA 1986; 83:4479-4483).

Since CD3-antibodies, bound to Fc receptors (FcRs) via their Fc-part, are exceedingly efficient in inducing T cell activation and cytokine release, it is of paramount importance to construct bispecific antibodies that are (i) Fc-depleted or - attenuated and (ii) directed to target cell associated antigens that are specifically expressed, that is, not expressed on normal cells not to be targeted. In this way "off-target" activation by (i) FcR expressing cells or (ii) by non-target cells carrying the target antigen is avoided- (Jung G and Müller-Eberhard HJ. An in vitro model for tumor immunotherapy with antibody-heteroconjugates. Immunol Today 1988; 9:257-260; Jung G, Freimann U, v.Marschall Z, Reisfeld RA and Wilmanns W. Target cell induced T cell activation with bi- and trispecific antibody molecules. Eur J Immunol 1991; 21:2431-2435).

The production of bispecific antibody molecules meeting this critical prerequisite in industrial quality and quantity remains a formidable challenge. Recently, a recombinant, bispecific single chain (bssc/BiTE) antibody molecule with CD19 X CD3 specificity, termed Blinatumomab, has demonstrated considerable efficiency in the treatment of patients with ALL (Bargou R, Leo E, Zugmaier G et al. Tumor regression in cancer patients by very low doses of a T cell-engaging antibody. Science 2008; 321:974-977) and is currently tested in phase III trials. Notably, the drug is applied as continuous 24-hour-infusion due to its low serum half-life. Safely applicable doses are approx. 50 µg per patient and day, which is 10.000 times lower than a single dose of an established monospecific anti-tumor antibody such as Rituximab (Adams GP, Weiner LM. Monoclonal antibody therapy of cancer. Nat Biotechnol. 2005; 23:1147-57). The resulting serum concentrations of Binatumomab are below 1 ng/ml (Topp MS, Kufer P, Gokbuget N et al. Targeted therapy with the T cell-engaging antibody blinatumomab of chemotherapy-refractory minimal residual disease in B-lineage acute lymphoblastic leukemia patients results in high response rate and prolonged leukemia-free survival. J Clin Oncol 2011; 29:2493-2498). This severe dose limitation that has also been observed in earlier clinical trials with different bispecific antibody molecules (Kroesen BJ, Buter J, Sleijfer DT et al. Phase I study of intravenously applied bispecific antibody in renal cell cancer patients receiving subcutaneous interleukin 2. Br J Cancer 1994; 70:652-661; Tibben JG, Boerman OC, Massuger LF et al. Pharmacokinetics, biodistribution and biological effects of intravenously administered bispecific monoclonal antibody OC/TR F(ab')2 in ovarian carcinoma patients. Int J Cancer 1996; 66:477-483) is due to off target T cell activation (unspecific T cell activation) resulting in systemic cytokine release. Obviously, this phenomenon prevents an optimal therapeutic activity of bispecific antibody molecules stimulating the TCR/CD3 complex.

In principle, dose limiting "off target" T cell activation by bispecific antibodies and the resulting toxicity problem may be caused by the following phenomena:
(P1) Binding via Fc-parts to FcR-positive cells. This can be avoided by using Fc-free or attenuated bispecific antibodies.
(P2) The target cell associated antigen targeted by the bispecific antibody molecule is not entirely target cell specific resulting in antibody molecule mediated T cell activation by normal cells that express and thus present target associated antigen. In a strict sense this is no "off target" activation, since it is still induced by antigen expressing target cells albeit the "wrong ones", namely by normal cells rather than e.g. malignant cells. Blinatumomab, the bispecific CD19 X CD3 antibody molecule mentioned above, certainly faces this problem since its target antigen CD19 is expressed on normal B lymphocytes.
(P3) "true" off-target T cell activation by the CD3 binding part of the antibody. This can occur if the antibody aggregates or multimerizes in solution. Under certain conditions, even a monovalent CD3 binding site within a bispecific antibody molecule is capable of inducing some unspecific T cell activation in the absence of target cells to which the antibody molecule specifically binds. In fact, this represents off target T cell activation in a strict sense, since cells carrying a target antigen or Fc receptors are not required to induce the phenomenon.

Thus, every bispecific antibody molecule containing an effector part that stimulates the T cell receptor (TCR)/CD3 complex faces this problem irrespective of its target specificity. For example, upon clinical application of bispecific TAA X CD3 antibody molecules, unspecific T cell activation may cause excessive cytokine release that, as mentioned above, severely limits safely applicable doses.

This unspecific T cell activation, however, is not only restricted to the use of bispecific antibody molecules but rather a phenomenon observed in all T cell engaging immunotherapies. For, example also the use of chimeric antigen receptor (CAR) modified T cells can lead to the so called cytokine release syndrome (Maus MV, Grupp SA, Porter DL, June CH. Antibody-modified T cells: CARs take the front seat for hematologic malignancies. Blood 2014; 123:2625-35). In one study, utilizing the autologous T cells modified with CARs based on the humanized monoclonal antibody trastuzumab (anti-ERBB2) and the CD28, 4-1 BB and CD3ζ signaling moieties, the patient died from a cytokine storm (Morgan RA1, Yang JC, Kitano M, Dudley ME, Laurencot CM, Rosenberg SA. Case report of a serious adverse event following the administration of T cells transduced with a chimeric antigen receptor recognizing ERBB2. Mol Ther 2010 Vol 18, No. 4, 843-851).

Therefore, due to these in some cases even fatal consequences, there is a need for reducing unspecific (unwanted) T cell activation upon T cell engaging therapies. This problem is solved by the embodiments reflected in the claims, described in the description, and illustrated in the Examples and Figures.

### SUMMARY OF THE INVENTION

The present invention relates to a blocking-reagent for use in reducing unspecific T cell activation in therapy, the therapy comprising administering to a subject a bispecific antibody molecule and/or a chimeric antigen receptor (CAR) modified T cell, wherein the bispecific antibody molecule comprises two binding sites
i) wherein the first binding site binds to an antigen associated with a target cell and
ii) wherein the second binding site binds to a T cell receptor (TCR)/CD3 complex, on an effector cell and/or
   wherein the CAR comprises
iii) an antibody molecule comprising a binding site that binds to an antigen associated with a target cell, and
iv) a TCR/CD3 signaling domain.

Thus, in other words, the present invention relates to a method of co-administering a blocking reagent (as defined herein) together with such a bispecific antibody molecule and/or such a chimeric antigen receptor (CAR) to reduce unspecific T cell activation mediated by the bispecific antibody molecule or the chimeric antigen receptor in therapy.

The present invention further relates to a pharmaceutical kit of parts, comprising in two separate parts:
a) a blocking-reagent, and
b) a bispecific antibody molecule,
   wherein the bispecific antibody molecule binds to
   i) a first antigen, and
   ii) a T cell receptor (TCR)/CD3 complex.

Also embraced by the present invention is a pharmaceutical kit of parts, comprising, in two separate parts:
a) a blocking-reagent, and
b) a chimeric antigen receptor (CAR) modified T cell
   wherein the CAR comprises
   i) an antibody molecule and
   ii) a TCR/CD3 signaling domain.

In addition, the present invention relates to an *in vitro* method for evaluating unspecific T cell activation, the method comprising
(i) contacting bystander cells and effector cells with bispecific antibody molecules as defined in herein that do not bind to the bystander cells, or
(ii) contacting bystander cells and effector cells with CAR T cells as defined herein that do not bind to the bystander cells, and
(iii) measuring unspecific T cell activation.

These aspects of the invention will be more fully understood in view of the following description, drawings and non-limiting examples.

### BRIEF DESCRIPTION OF THE DRAWINGS

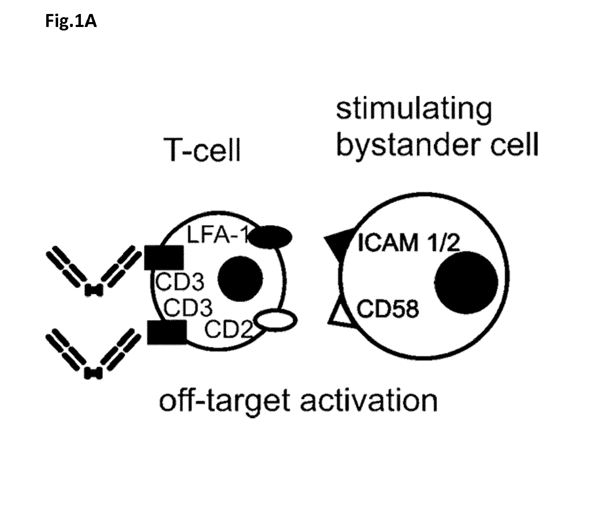

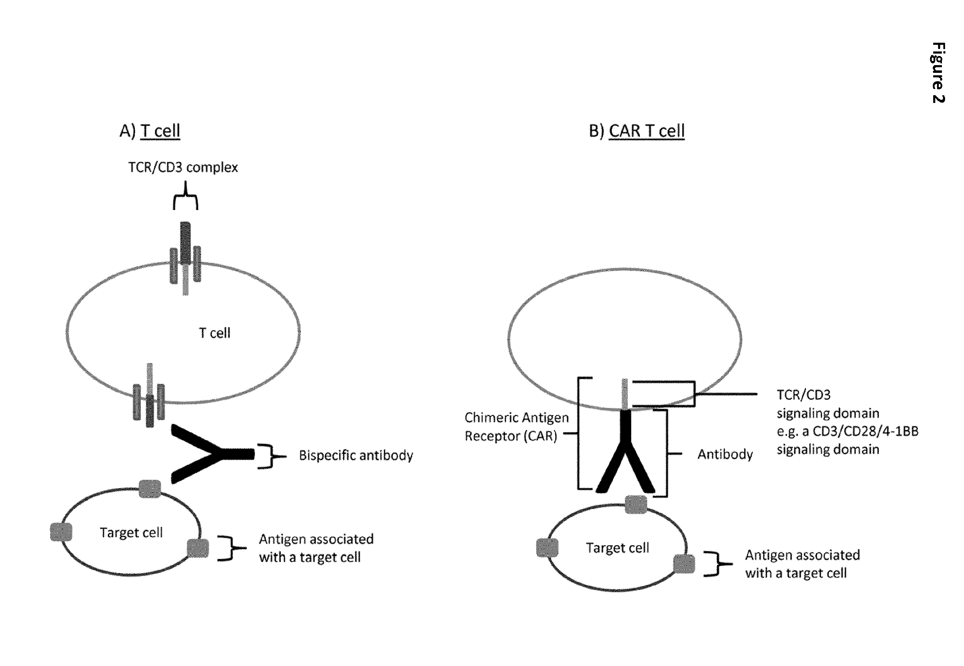

**Fig.1A** is a schematic representation of the mechanism by which it is believed that stimulating bystander cells cause unwanted and unspecific T cell activation in therapy as explained here, in which a bispecific antibody molecule and/or a chimeric antigen receptor (CAR) modified T cell is administered to a patient. While this activation mechanism is illustrated in Fig. 1A using a bispecific antibody molecule, due to the structural similarities with CAR T cells (see Fig. 2), it is believed that the same mechanism applies to the unspecific T cell activation that has been observed in therapeutic administration of CAR T cells. As shown in Fig. 1A, the bispecific antibody molecule comprises two binding sites. The first binding site binds to an antigen associated with a target cell (targeting part) and the second binding site binds to the T cell receptor (TCR)/CD3 complex on a T cell serving as effector cell (effector part of the molecule). Such a bispecific antibody molecule, when bound to T cells via CD3 may induce T cell activation in the absence of target cells (to which they may bind with via their targeting part) if stimulating bystander cells (SBCs) are present. Lymphoid- and/or vascular endothelial cells carrying adhesion and co-stimulatory molecules, such as ICAM and CD58, may assume the role of SBCs. As indicated in Fig. 1A, for example, CD58 that is present on the SBC (for example, on macrophage or antigen presenting cells, also known as APCs) can bind to its natural ligand CD2 on the T cell. If the T cell has bound to it a bispecific antibody (via the CD3-binding effector part of this molecule), the binding of CD58, expressed on an APC, to CD2 can provide a co-stimulatory signal for T cell activation, in this time unwanted, since unspecific T cell activation results, that is, activation on the absence of the target cells to which the bispecific antibody may bind via its targeting part. Similarly, the cell adhesion molecules ICAM-1 or ICAM-2, present on the SBCs, can bind to its ligand LFA-1, thereby providing the co-stimulatory signal that leads to unspecific T cell activation. It is believed that the engagement of LFA-1 or other integrins containing CD18 by the respective ligands expressed on SBCs, is a necessary but not sufficient condition for the emergence of the SBC effect. As shown herein, blocking for example, CD18, a component of LFA-1 and other integrins, by means of a CD18 blocking-reagent as explained herein abolishes this type of "off-target" activation caused by bispecific antibody molecules or CAR T cells.

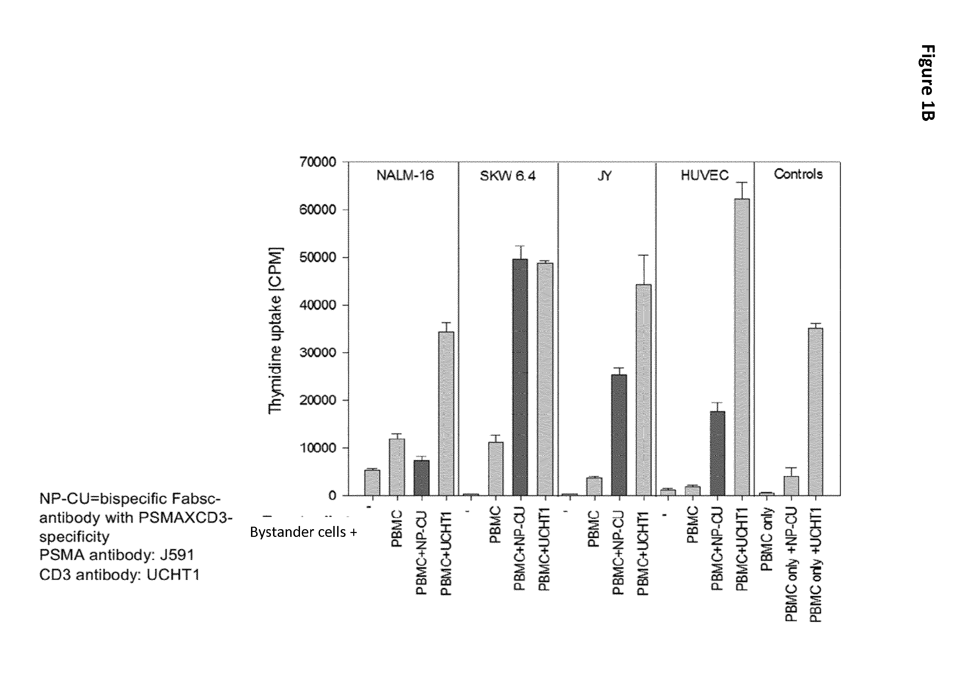

**Fig. 1B** shows that stimulating bystanders cells (SBCs) induce the activation of peripheral blood mononuclear cells (PBMCs) together with bispecific Fabsc-antibody molecules in the absence of target cells. PBMCs were seeded together with the different indicated bystander cells (SBCs). Bystander cells were irradiated, otherwise their proliferation would interfere with that of the T cells. These "co-cultures" were compared to control PMBC cultures not comprising the irradiated bystander cells. In a next step a bispecific "Fabsc"-antibody molecule as described in International patent application WO 2013/092001 comprising a single chain Fv fragment which is connected to an Fab fragment via a CH2 domain with PSMA X CD3 specificity was added to these different cell cultures (indicated as "PBMC+NP-CU" on the y-axis). The single chain Fv fragment of this PSMA x CD3 bispecific "Fabsc"-antibody molecule binds to CD3, while the Fab fragment of this antibody molecule binds to PSMA. Notably, the prostate specific membrane antigen (PSMA) of the bispecific antibody molecule is neither expressed on PBMCs nor on SBCs. Thus, the PSMA x CD3 antibody molecule did not bind to any target cell present in the PMBC-SBC co-culture - the co-culture therefore lacked any target cells. It follows that an off target (unspecific) T cell is measured with this experiment activation.

As a positive control, an anti-CD3 antibody molecule was added to different wells containing the co-cultures (indicated as "PBMC+UCHT1" on the y-axis). It is well established in the field that an intact CD3 antibody binds to FcR expressing monocytes within the PBMC via its Fc-part, thereby inducing maximal activation of the T cells contained in the PBMC culture. In addition, also cultures comprising only PBMC (indicated as "PBMC" on the y-axis) or only SBCs (NALM-16, SKW 6.4, JY or HUVEC cells, indicated as "-" on the y-axis, were analyzed for thymidine uptake. The dark grey bars indicate the effect of the bispecific antibody molecule.

Conclusion: The intact bivalent CD3 antibody molecule led to n FcR-dependent maximal T cell activation and served as a positive control. Cultures comprising only one cell type (PBMC or SBC cells) or both cell types in the absence of the antibody did not resume a high amount of cell proliferation. Contrary thereto, the bispecific antibody molecule increased proliferation in cultures containing PBMC and SKW6.4- and JY lymphoblastoid cells as well as human umbilical vein endothelial cells (HUVECs). However, proliferation was not increased in the control culture or in the co-culture of lymphoblastoid NALM16 cells and PBMC. Thus, unspecific T cell activation mediated by bispecific antibody molecules could be promoted by certain bystander cells such as SKW 6.4, JY and HUVEC cells.

**Fig.2** shows an exemplary comparison between bispecific antibody molecule therapy and modified CAR T cell therapy. It illustrates that, in both cases, an antibody defined stimulation of the T cell receptor (TCR)/CD3 complex takes place, the antibody being directed to an antigen on a target cell. Thus a, CAR T transfected with a chimeric receptor, consisting of an antibody and a CD3 signaling domain (B), can be considered as being functionally equivalent to a T cell with a bispecific antibody irreversibly fixed to its surface (A).

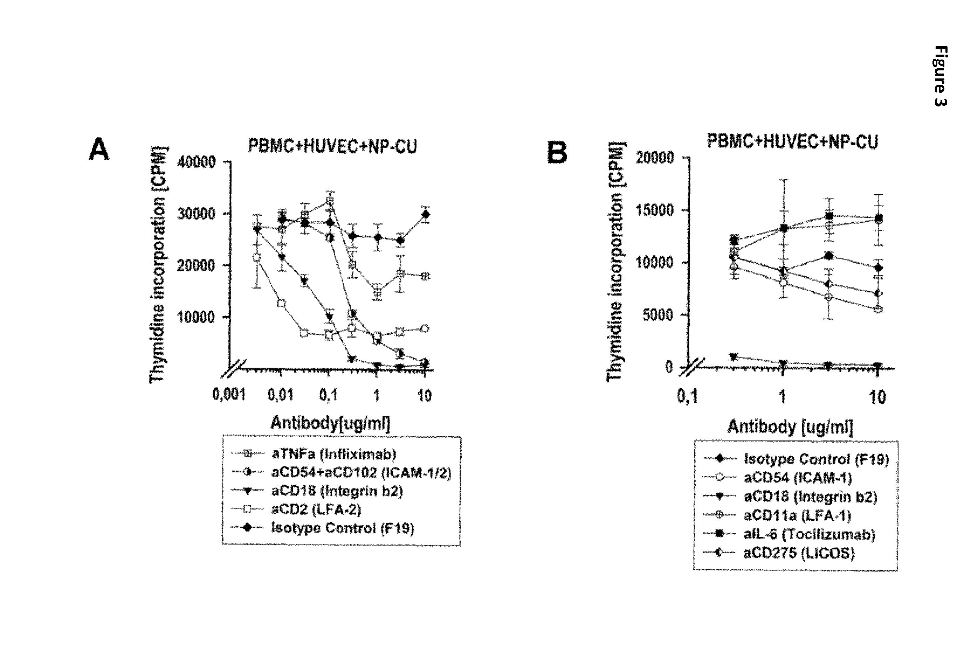

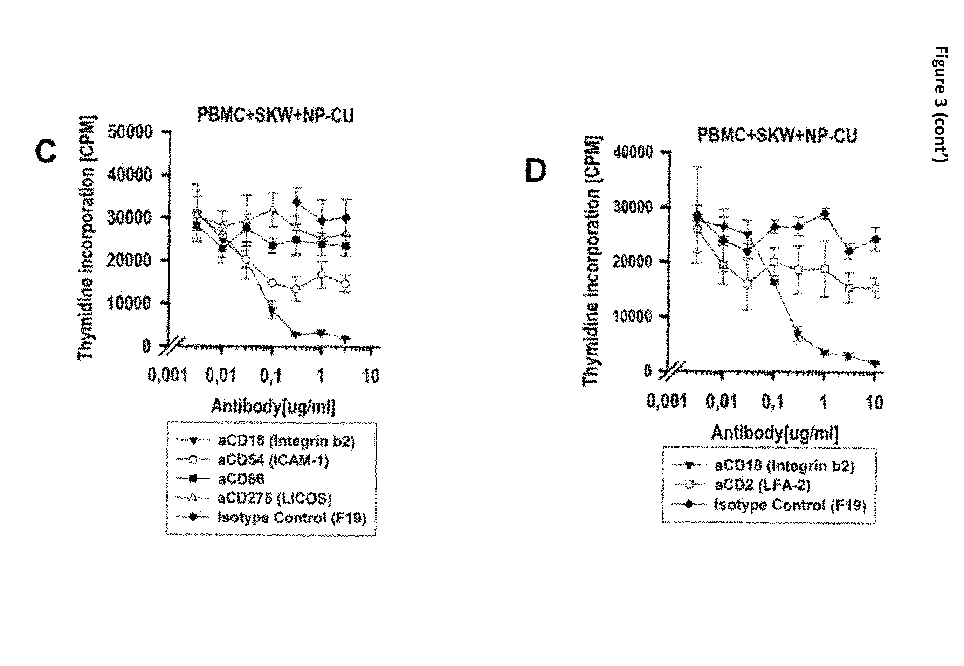

**Fig.3** shows that various antibody molecules (blocking-reagents) inhibit activation of PBMC (T cells) with bispecific antibody molecules and SBCs. The experimental set up is as set forth in the description of Fig. 1B and Example 1. In addition to this set up, now blocking-reagents to various adhesion molecules and cytokines were added to the PBMC-SBC co-cultures. Therefore, with this experimental set-up, the influence of a blocking-reagent on off target cell activation was measured. Due to the design of the experiment the bispecific antibody molecule could stimulate effector cells, which comprised the TCR/CD3 receptor. This receptor is expressed by T cells. Thus, a decrease in proliferation (compared to the isotype control) indicated that unspecific T cell activation is reduced.

The SBCs used in **Fig. 3 (A)** were irradiated human umbilical vein endothelial cells (HUVECs). The addition of the control showed the amount of base-line cell proliferation. The addition of anti-TNFalpha antibody molecules resulted in a slight decrease in proliferation. Notably, the addition of a combination of anti-CD54 with anti-CD102 antibody molecules, anti-CD18 antibody molecules or anti-CD2 antibody molecule resulted in a marked decrease in cell proliferation (Figure 3A).

The SBCs used in **Fig. 3 (B)** were again irradiated human umbilical vein endothelial cells (HUVECs). The addition of the control indicated the amount of base-line cell proliferation. Provision of an anti-IL-6R antibody molecule or an anti-CD11a antibody molecule showed an increase in cell proliferation, while the addition of an anti-CD275 antibody molecule or an anti-CD54 antibody molecule resulted in a moderate decrease of proliferation. Notably, adding an anti-CD18 antibody molecule resulted in a complete block of proliferation.

The SBCs used in **Fig. 3 (C)** were irradiated SKW cells. The addition of the control again showed the amount of base-line cell proliferation for this experiment. The provision of an anti-CD275 antibody molecule or an anti-CD86 antibody molecule showed a slight decrease in cell proliferation, while the addition of an anti-CD54 molecule resulted in a marked decrease in proliferation. Notably, the addition of an anti-CD18 antibody molecule resulted in an almost complete block of proliferation.

The SBCs used in **Fig. 3 (D)** were again irradiated SKW cells. Here, the isotype control showed a proliferation of about 25000-30000 cpm, while the addition of an anti-CD2 antibody molecule resulted in a slight decrease in proliferation. Again, the provision of an anti-CD18 antibody molecule showed an almost complete block of proliferation.

Conclusion: Antibody molecules binding to CD54, CD2, ICAM 1, ICAM1 and 2, LICOS and TNFa moderately inhibited the T cell activation mediated by bispecific antibody molecules and SBCs. The CD18 antibody molecule TS 1/18 completely blocked unspecific T cell activation.

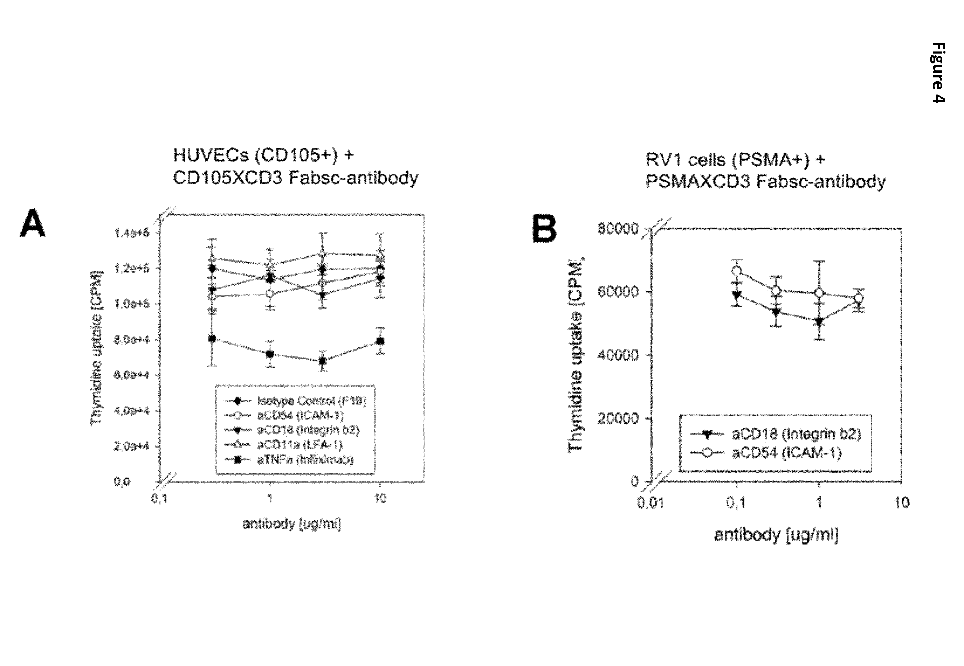

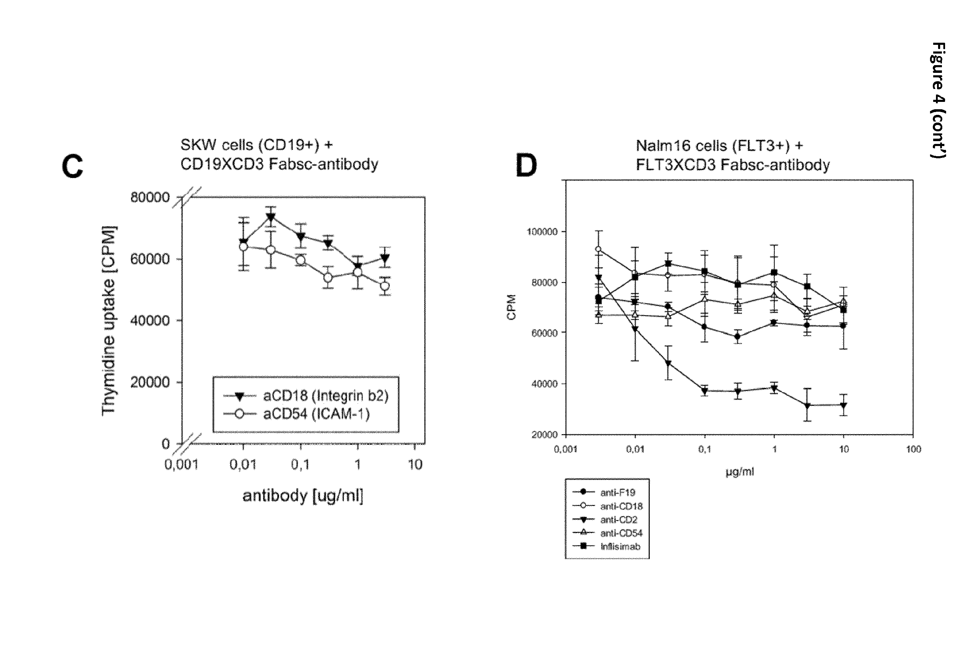

**Fig. 4** shows that a CD18 antibody molecule (acting as blocking-reagent) does not block the activity of bispecific antibody molecules in the presence of target cells. Also in Fig. 4 the experimental set up was identical to that described in Example 1 and Fig. 1B except that the bispecific Fabsc antibody molecule added recognized a target antigen expressed on the SBCs. Thus, different bispecific antibody molecules were added to the different co-cultures. These bispecific antibody molecules (which all have the format described in International patent application WO 2013/092001), namely CD105xCD3 (A), PSMA X CD3 (B), CD19 X CD3 (C) and FLT3XCD3 (D) antibody molecules, bound to the respective bystander cells present in each co-culture, that is, endoglin (CD105) in the case of the HUVEC cells, CD19 in the case of the SKW cells, PSMA in the case of the RV1 cells and FLT3 in the case of the NALM16 cells.

Furthermore, in each co-culture the effect of different blocking-reagents that were shown to reduce the off target T cell activation (in Example 2) were analyzed. In contrast, to the experiments shown in Figure 1B and 3, in Figure 4 the on-target activation of cells is depicted. This means that in this case only blocking-reagents that do not block the "on target cell" proliferation (T cell activation) (Fig. 4), but do block "off target" cell proliferation (T cell activation) (Fig. 3) are the most interesting blocking-reagents for the purposes of the present invention.

As can be seen in **Fig. 4(A)** the anti-CD54 antibody molecule, the anti-CD18 antibody molecule and the anti-CD11 a antibody molecule performed equally to the isotype control. Thus, these blocking-reagents did not have an effect on the proliferation (T cell activation). On the contrary, the addition of the anti-TNFa-antibody molecule decreased the proliferation of T cells (and therefore also the specific T cell activation) in this experiment.

In **Fig. 4(B)** and **Fig. 4(C)** the blocking-reagents, namely the anti-CD54 antibody molecule and the anti-CD18 antibody molecule had similar effects on proliferation in the presence of different bystander cells.

Notably, in **Fig. 4(D)** the anti-CD18 antibody molecule, the anti-CD54 antibody molecule, and the anti-TNFalpha antibody molecule all did not influence the proliferation notably different from the isotype control. Only the anti-CD2 antibody molecule decreased the proliferation.

Conclusion: In the presence of the respective CD105 x CD3-, PSMA x CD3-, CD19 x CD3- and FLT3 x CD3-antibody molecules recognizing target antigens on the respective SBCs, the activation of PBMCs was not inhibited by antibody molecules binding to CD18, LFA-1 and CD54. It was moderately affected by an antibody molecule to TNF and markedly inhibited by the CD2 antibody molecule used.

Thus, especially antibody molecules comprising binding domains binding to CD18, LFA-1 and CD54 did not effect on target T cell activation of bispecific antibody molecules. However, as shown in Figure 3, these blocking-reagents could at the same time reduce unspecific T cell activation. Therefore, such blocking-reagents are particularly well suited for the purposes of the present invention.

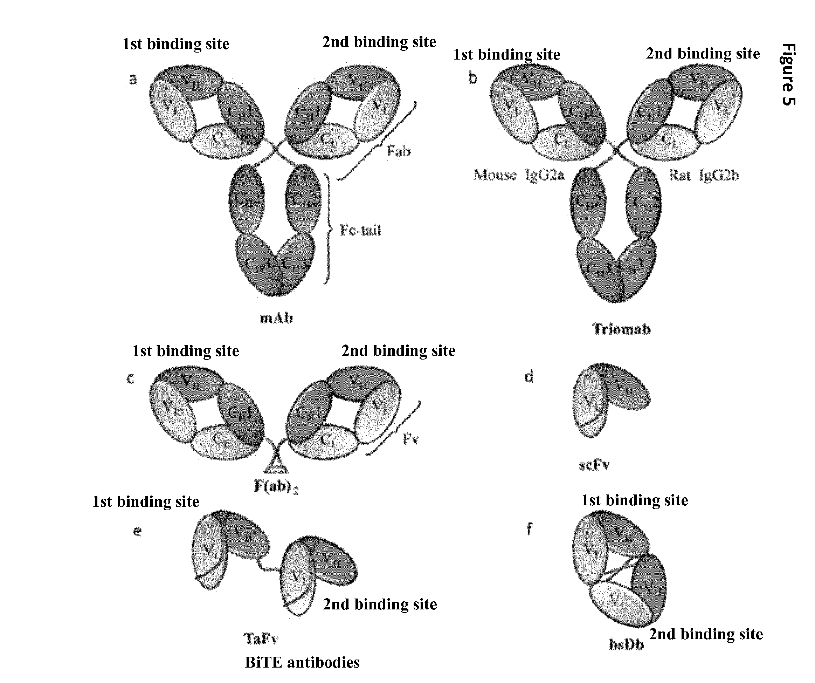

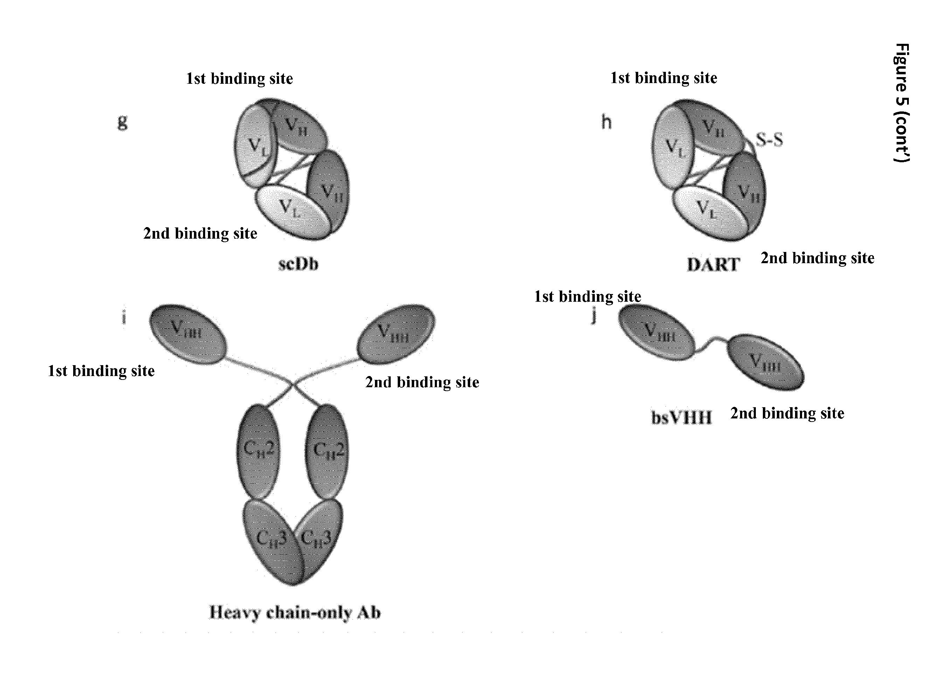

**Fig. 5** The blocking-reagent used in the present invention as well as the bispecific antibody molecule or antibody molecule of the CAR of the CAR modified T cell used in therapy in accordance with the present invention can be present in different bispecific antibody molecule formats. Different exemplary bispecific antibody molecules formats are shown in this Figure. Variable heavy chain domains (VH) are depicted in dark grey, variable light chain domains (VL) are depicted in light grey. The different specificities are indicated for each bispecific molecule. Peptide linkers are shown as gray lines. **Fig.5 (a)** mAb, monoclonal antibody; **(b)** Triomab, bispecific rat/mouse antibody; **(c)** F(ab)2, two chemically crosslinked fragment antigen binding (Fab) regions; **(d)** scFv, single chain variable fragment; **(e)** TaFv, tandem single chain variable fragment, also termed BiTE antibodies; **(f)** bsDb, bispecific diabody; **(g)** scDb, single chain diabody; **(h)** DART, dual affinity retargeting molecule; **(i)** Heavy chain-only antibody; **(j)** bsVHH, bispecific variable domains of heavy chain-only Ab. This Figure was modified from Roeland Lamerisa, Renée C.G. de Bruina, Famke L. Schneidersa, Paul M.P. van Bergen en Henegouwenb, Henk M.W. Verheula, Tanja D. de Gruijla, Hans J. van der Vliet Bispecific antibody platforms for cancer immunotherapy. Crit Rev Oncol Hematol. 2014 Aug 20. pii: S1040-8428(14)00135-8. Other possible bispecific antibody molecules that can be used in the T cell engaging therapies disclosed herein are the "Fabsc"-antibody molecules that have been used in the Examples of the present invention and that are described in International patent application WO 2013/092001.

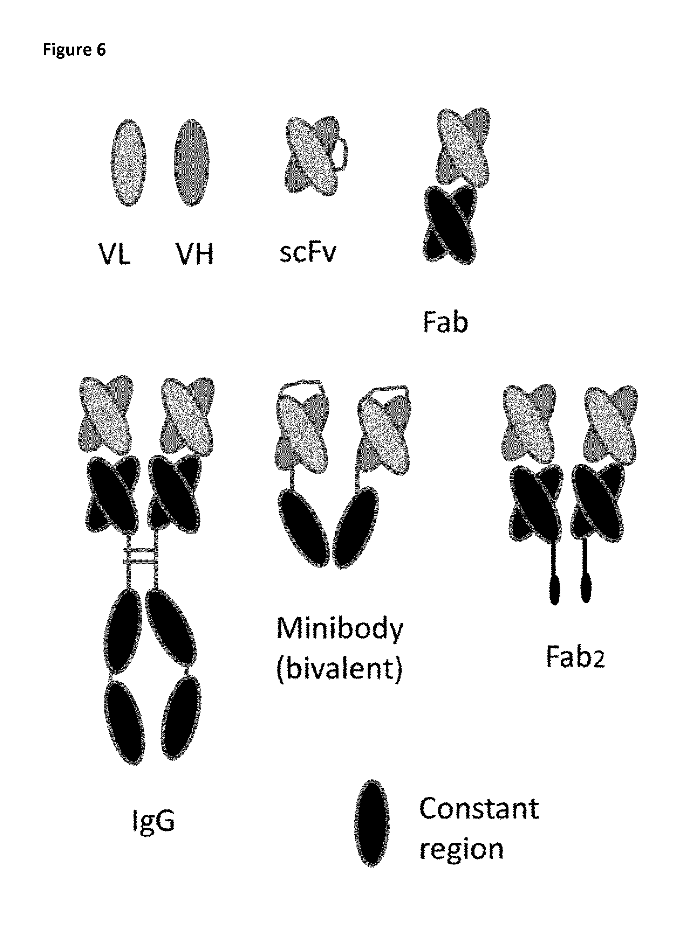

**Fig. 6** shows different exemplary formats of antibody molecules. The blocking-reagent used in the present invention as well as the antibody molecule of the CAR of the CAR modified T cell used in therapy in accordance with the present invention can be present in any of the depicted different antibody molecule formats. For blocking antibodies, the use of an attenuated Fc-part is preferred in order to avoid the killing of the cells targeted.

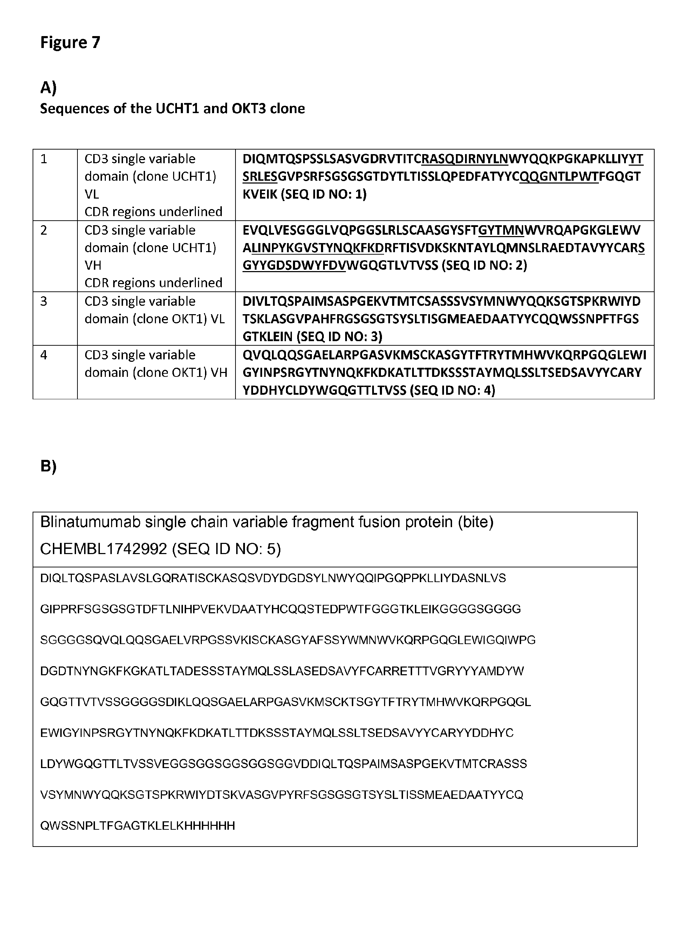

**Fig. 7 (A)** depicts the amino acid sequences of the variable domains of the antibodies UCHT1 and OKT3. **Fig. 7 (B)** depicts the amino acid sequence of the bispecific single chain antibody molecule Blinatumumab (CHEMBL1742992, SEQ ID NO: 5).

### DETAILED DESCRIPTION

The present invention provides a novel approach to reduce off target T cell activation (unspecific T cell activation) in therapeutic applications by using blocking-reagents. Without wishing to be bound to theory, it is believed that these blocking-reagents reduce unspecific T cell activation by inhibiting or decreasing *in vivo* the interaction between the T cells and bystander cells of the patient that is being treated with either a bispecific antibody molecule that binds to a T cell receptor (TCR)/CD3 complex (on an effector cell) and/or a CAR T cell that carries a TCR/CD3 signaling domain within a transfected chimeric receptor (cf. Fig.1A and Fig. 2, respectively). Such a bystander cell may be any cell that is capable of supporting T cell activation. In addition, the present invention relates to the use of blocking-reagents in reducing side-effects of bispecific antibody therapy and CAR T cell therapy.

Unwanted side-effects are frequently observed in these two types of T cell engaging therapies (administration of a bispecific antibody molecule that binds to a T cell receptor or a CAR T cell that binds to a TCR/CD3 signaling domain on an effector cell). In more detail, in bispecific antibody molecule therapy strong immune cell responses such as elevated cytokine releases (cytokine release syndrome) or even cytokine storms have been frequently observed. To circumvent these negative effects slow infusions of low concentrations of bispecific antibody molecules have been used to lessen this problem. For example, it has been shown that blinatumomab, when administered at low doses by continuous infusion to patients with B-lineage acute lymphoblastic leukemia, resulted in only a transient release of cytokines (Klinger M, Brandl C, Zugmaier G, Hijazi Y, Bargou RC, Topp MS, Gökbuget N, Neumann S, Goebeler M, Viardot A, Stelljes M, Brüggemann M, Hoelzer D, Degenhard E, Nagorsen D, Baeuerle PA, Wolf A, Kufer P. Immunopharmacologic response of patients with B-lineage acute lymphoblastic leukemia to continuous infusion of T cell-engaging CD19/CD3-bispecific BiTE antibody blinatumomab. Blood 2012; 119:6226-33). However, in this way the application is limited to these continuous infusions of the bispecific antibody molecule, which are not always practical in the clinic. More importantly, the rather low doses applicable are not sufficient to exert optimal therapeutic activity.

Similarly to the bispecific antibody molecule therapy, also CAR modified T cell therapy is accompanied with unwanted side-effects. Upon application of autologous T cells expressing a CD19-specific CD28/CD3ζ dual signaling chimeric antigen receptor (termed 19-28z) undesired cytokine elevations were observed (Brentjens RJ, Davila ML, Riviere I, Park J, Wang X, Cowell LG, Bartido S, Stefanski J, Taylor C, Olszewska M, Borquez-Ojeda O, Qu J, Wasielewska T, He Q, Bernal Y, Rijo IV, Hedvat C, Kobos R, Curran K, Steinherz P, Jurcic J, Rosenblat T, Maslak P, Frattini M, Sadelain M. CD19-targeted T cells rapidly induce molecular remissions in adults with chemotherapy-refractory acute lymphoblastic leukemia. Sci Transl Med 2013; 5:1-9). In another study, autologous T cells were modified with CARs based on the humanized monoclonal antibody trastuzumab and the CD28, 4-1 BB and CD3ζ signaling moieties. These CAR modified T cells were administered to a patient with cancer, who then suffered from a cytokine storm (Morgan et al., (2010) cited herein).

The fact that the bispecific antibody molecule therapy as well as the CAR modified T cell therapy can lead to similar side-effects can be explained by the structural similarity between bispecific antibody molecules and CAR modified T cells. This structural similarity is also depicted in **Fig. 2****.** Notably, the observed cytokine release syndromes or cytokine storms observed in these two T cell engaging therapies are thought to be due to a high level of immune activation (Lee DW, Gardner R, Porter DL, Louis CU, Ahmed N, Jensen M, Grupp SA, Mackall CL. Current concepts in the diagnosis and management of cytokine release syndrome. Blood. 2014 Jul 10; 124(2):188-95). This high level of immune activation could be mediated (at least partially) by unspecific T cell activation.

The present invention provides a way to reduce such unspecific T cell activation or side-effects by using blocking-reagents which are co-administered together with bispecific antibody molecules or modified CAR T cells. More specifically, the present invention relates to a blocking-reagent that is used in reducing unspecific T cell activation in therapy, the therapy comprising administering to a subject a bispecific antibody molecule and/or a chimeric antigen receptor (CAR) modified T cell,
wherein the bispecific antibody molecule comprises two binding sites
i) wherein the first binding site binds to an antigen associated with a target cell and
ii) wherein the second binding site binds to a T cell receptor (TCR)/CD3 complex, on an effector cell and/or
   wherein the CAR comprises
iii)an antibody molecule comprising a binding site that binds to an antigen associated with a target cell, and
iv)a TCR/CD3 signaling domain.

As outlined above both, the bispecific antibody molecule and the CAR modified T cell, comprise an antibody molecule comprising a binding site that binds to an antigen associated with a target cell. Such a "target cell" can be any cell to which the first binding site of a bispecific antibody molecule binds or to which the modified CAR of a CAR modified T cell binds. Therefore, the target cell, to which these T cell engaging therapies are directed, has to express the target cell associated antigen. In some embodiments, the target cell expresses a tumor associated antigen (TAA) or an antigen associated with an autoimmune disease.

The term "tumor associated antigen" as used herein refers to an antigen that is or can be presented on a surface that is located tumor cells. These antigens can be presented on the cell surface with an extracellular part, which is often combined with a transmembrane and cytoplasmic part of the molecule. These antigens can in some embodiments be presented only by tumor cells and not by normal, i.e. non-tumor cells. Tumor antigens can be exclusively expressed on tumor cells or may represent a tumor specific mutation compared to non-tumor cells. In such an embodiment, a respective antigen may be referred to as a tumor-specific antigen. Some antigens are presented by both tumor cells and non-tumor cells, which may be referred to as tumor associated antigens. These tumor associated antigens can be overexpressed on tumor cells when compared to non-tumor cells or are accessible for antibody binding in tumor cells due to the less compact structure of the tumor tissue compared to non-tumor tissue. In some embodiments, the tumor associated surface antigen is located on the vasculature of a tumor.

In some embodiments, the target cell expresses an TAA that is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1R, (CD115), IL-3R (CD123), CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate blocking protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, Tie2, mesothelin, epithelial glycoprotein 2 (EGP2), epithelial glycoprotein 40 (EGP40), cancer antigen 72-4 (CA72-4), interleukin 13 receptor alpha-2 subunit, IL13Rα2, Ig kappa light chain (κ), GD3-ganglioside (GD3), GD2-ganglioside (GD2), CD171, NCAM, alpha folate receptor (αFR), Lewis(Y), fetal acetylcholine receptor (FAR), avian erythroblastic leukemia viral oncogene homolog 3 (ERBB3), avian erythroblastic leukemia viral oncogene homolog 4 (ERBB4), avian erythroblastic leukemia viral oncogene homolog 2 (ERBB2), hepatocyte growth factor receptor (HGFR/c-Met), claudin 18.2, claudin 3, claudin 4, claudin 1, claudin 12, claudin 2, claudin 5, claudin 8, claudin 7 and CD138. The TAA can also be one of CD19, CD20, CD30, CD33, CD138, Lewis Y, EGFR and Ig kappa light chain (κ).

Accordingly, the target cell can be a cancer or tumor cell. A "tumor cell" or "cancer cell" is a cell that abnormally divides. In particular, these cells grow and divide at an unregulated, quickened pace. However, a tumor cell in the sense of the present invention does also include leukemia cells, and carcinoma cells in situ. A tumor cell can be benign, pre-malignant, or malignant. In a preferred embodiment, the tumor cells are pre-malignant or malignant. Most preferably, the tumor cell is malignant.

A target cell may alternatively express an antigen, which is expressed by a cell which is associated with or mediates autoimmune diseases. An "autoimmune disease" occurs when a specific adaptive immune response is mounted against self-antigens. The normal consequence of an adaptive immune response against a foreign antigen is the clearance of the antigen from the body. For example, virus-infected cells are destroyed by cytotoxic T cells, while soluble antigens are cleared by formation of immune complexes of antibody and antigen, which are taken up by cells of the mononuclear phagocytic system such as macrophages. When an adaptive immune response develops against self-antigens, it is usually impossible for immune effector mechanisms to eliminate the antigen completely. In this way, a sustained immune response occurs, which may cause chronic inflammatory injury to tissues or which may prove lethal.

Such an antigen, which is associated with an autoimmune disease can be any antigen which is present on the cell surface of a cell or in the extracellular matrix associated with a cell that mediates (or is associated with) an autoimmune disease. An antigen associated with an autoimmune disease can also be specifically expressed by a cell that mediates an autoimmune disease. Exemplary antigens, which are associated with autoimmune diseases, include α4 subunit of α4β1 and α4β7 integrin, α4β7 integrin, BAFF, CD2, CD3, CD19, CD20, CD22, CD52, CD80 and CD86.

A bispecific antibody molecule that is used in therapy in accordance with the present invention thus binds with its first binding site to an antigen associated with a target cell as described above. In addition, with its second binding site the bispecific antibody molecule binds to the TCR/CD3 complex on an effector cell.

A suitable "effector cell" can be any cell that is capable of killing other cells. An effector cell, in accordance with the present invention, expresses the T cell receptor (TCR)/CD3 complex. Illustrative examples of such effector cells are T cells that carry the αβ- or the γδ-receptor, cytotoxic T cells or T helper cells.

A "T cell receptor" (TCR) allows a T cell to bind to and, if additional signals are present, to be activated by and respond to an antigen presented by another cell called the antigen-presenting cell or APC. The T cell receptor is known to resemble a Fab fragment of a naturally occurring immunoglobulin. It is generally monovalent, encompassing α- and β-chains, in some embodiments it encompasses γ-chains and δ-chains (supra). Accordingly, in some embodiments the TCR is TCR (alpha/beta) and in some embodiments it is TCR (gamma/delta). The T cell receptor forms a complex with the CD3 T cell signaling unit. CD3 is a protein complex and is composed of four distinct chains. In mammals, the complex contains a CD3γ chain, a CD3δ chain, and two CD3ε chains. These chains associate with a molecule known as the T cell receptor (TCR) and the ζ-chain to generate activation signal in T lymphocytes. Hence, the T cell specific receptor forms a complex with the CD3 signaling unit (TCR/CD3 complex).

By simultaneously binding to the TCR/CD3 complex on an effector cell and an antigen associated with a target cell, the bispecific antibody molecule brings these two cells into close contact. Without wishing to be bound to theory it is presumed that by this close contact, the effector cell is (specifically) activated and kills the target cell. This mechanism is also called "target cell restricted T cell activation".

However, as shown by the present invention, such an activation of effector cells, which are T cells due to their expression of the TCR/CDR complex, can also occur in an unspecific way. An "unspecific T cell activation" or an "off target activation" of T cells could be any activation of T cells, which is not related to a target cell-restricted activation of T cells (effector cells) upon bispecific antibody molecule and/or CAR modified T cell therapy. For example, an off target T cell activation could thus be a target cell-independent T cell activation. An unspecific T cell activation can also comprise the binding of second binding site of the bispecific antibody molecule to a T cell receptor (TCR)/CD3 complex on a T cell (effector cell), wherein the first binding site of the bispecific antibody molecule does not bind to an antigen associated with a target cell. In other embodiments, the unspecific T cell activation comprises the activation of effector cells in the absence of target cells.

An unspecific T cell activation can also comprise that non-target cells such as bystander cells activate T cells. Such a "non-target cell" does not express the antigen to which the first binding site of the bispecific antibody molecule binds. Likewise, such a "non-target cell" does not express the antigen to which the modified CAR binds. In general, the non-target cell may be a lymphocyte, a monocyte, a macrophage or an endothelial cell. In one embodiment, the non-target cell is a bystander cell.

A "bystander cell" as used herein is any cell supporting or increasing unspecific T cell activation. It can be any body cell including but not limited to an endothelial cell or a lymphatic cell that is capable of supporting T cell activation together with a soluble, monomeric molecule binding to the TCR/CD3 complex. Such a soluble or monomeric molecule binding to the TCR/CD3 complex can be the bispecific antibody molecule. In the case of CAR T cells the chimeric molecule has been irreversibly transfected into T cells (see Figure 2).

Under physiological (*in vivo*) conditions bystander cells can, for example, be endothelial cells and lymphatic cells in the lymph node compartment. That fact the interaction with endothelial cells contributes to T cell activation by bispecific TAAXCD3 Fab₂ antibody molecules has already been demonstrated by Molema et al. (Molema G, Tervaert JW, Kroesen BJ, Helfrich W, Meijer DK, de Leij LF. CD3 directed bispecific antibodies induce increased lymphocyte-endothelial cell interactions in vitro. Br J Cancer 2000; 82:472-479). Transendothelial migration of T cells during *in vivo* administration of bispecific antibody molecules is also suggested by the rapid - albeit transient - lymphocyte depletion observed during treatment (Klinger et al., (2012) cited above). This phenomenon most likely contributes significantly to unspecific T cell activation induced by bispecific antibody molecules. Thus, in some embodiments, the bystander cell is an endothelial cell or a lymphatic cell. In other embodiments, the bystander cell is a non-target cell. Therefore, in one embodiment, the blocking-reagent used in the present invention reduces the activation of effector cells caused by a bystander cell.

Without wishing to be bound to theory it is believed that blocking-reagents used in the present invention are effective in reducing unspecific T cell activation by interfering with/blocking the interaction of T cells and bystander cells. To achieve this interference/blocking, the blocking-reagent used in the present invention can, for example, bind to a cell adhesion molecule (present on the cell surface of such a bystander cell) or a cytokine (this cytokine can be a secretory protein and is not necessarily present on the surface of a bystander cell). These two classes of molecules (cell adhesion molecule or a cytokine, also referred herein as "target molecules") usually mediate cell-cell "communications" or "interactions". Thus, by binding to at least one of such target molecules, a blocking-reagent used in the present invention can reduce cell-cell interactions and in particular interactions between effector cells and bystander cells.

A "cell adhesion molecule" is a protein located on the cell surface, which is involved in binding with other cells or with the extracellular matrix (ECM). Examples of cell adhesion molecules include membrane proteins such as cadherines, N-CAMs, mucin-like CAMs or integrins. In some embodiments, the blocking-reagent used in the present invention binds a cell adhesion molecule that is selected the group consisting of CD18, CD11a, CD11b, CD11c, ICAM-1 (CD54), ICAM-2 (CD102), LFA1, LFA2 (CD2), CD58, CD86, CD80, OX-40 (CD134), 4-1BB and/or LICOS (CD275). Particularly suitable are CD18, CD54, CD58, CD102, CD2, CD86 and CD275.

"Cytokines" are small proteins (∼25 kDa) that are released by various cells in the body, usually in response to an activating stimulus, and induce responses through binding to specific receptors. Non-limiting examples of cytokines include interleukin-1 (IL-1), interleukin-5 (IL-5), interleukin-6 (IL-6), interleukin-10 (IL-10), interleukin-12 (IL-12), interleukin-13 (IL-13), TNF-alpha, interferon alpha, interferon beta, interferon gamma and the chemokine interleukin-8 (IL-8). In some embodiments, the blocking-reagent used in the present invention binds to TNFalpha. In general, cytokines are present in a soluble form. Alternatively, as for example for the TNF-alpha, cytokines can also be present in a membrane-bound form. Therefore, a blocking-reagent used in the present invention can bind a soluble cytokine and/or a membrane-bound cytokine.

Since a blocking-reagent used in the present invention can bind to a cell adhesion molecule or a cytokine (soluble/membrane bound), in addition to sterically block the interaction of such molecules with effector cells the blocking-reagent can also interfere with the function of these molecules. Thus, a blocking-reagent used in the present invention can, for example, interfere with cytokine function by binding to soluble or insoluble cytokine receptors. Examples of such receptors are receptors of the hematopoietin-receptor family (class I cytokine receptor family), the class II cytokine receptor superfamily, the tumor necrosis factor-receptor (TNFR) family, and the chemokine-receptor family. A blocking-reagent used in the present invention can also interfere with the binding of cell adhesion molecules to their binding partner(s). One way to achieve to interfere with the binding of cell adhesion molecule can be by binding of the blocking-reagent to such a binding partner. Such binding partners can, for example, be fibronectin or laminin or other matrix molecules.

As described above, the blocking-reagent used in the present invention can be capable of binding to certain cell adhesion molecules or cytokines. Therefore, a blocking-reagent may be any molecule that comprises a binding site that is able to bind to a cell adhesion molecule, cytokine, cytokine receptor, laminin, fibronectin or other extracellular matrix molecules. In some embodiments, the blocking-reagent is selected from the group consisting of a (full-length complete) antibody, an antibody fragment (for example, a divalent antibody fragment or a monovalent antibody fragment) or a proteinaceous binding molecule with antibody-like binding properties.

Such an "antibody" can be, as indicated above, a full length antibody, a recombinant antibody molecule, or a fully human antibody molecule. A full length antibody is any naturally occurring antibody. The term "antibody" also includes immunoglobulins (Ig's) of different classes (i.e. IgA, IgG, IgM, IgD and IgE) and subclasses (such as IgG1, IgG2 etc.). Such full length antibodies can be isolated from different animals such as e.g. different mammalian species. The "recombinant antibody molecule" refers to an antibody molecule, the genes of which have been cloned, and is produced recombinantly in a host cell or organism, using well-known methodologies of genetic engineering. Typically, a recombinant antibody molecule been genetically altered to comprise an amino acid sequence, which is not found in nature. Thus, a recombinant antibody molecule can be a chimeric antibody molecule or a humanized antibody molecule.

The blocking-reagent used in the present invention can also be an "antibody fragment". Such antibody fragments comprise any part of an antibody, which comprises a binding site. Illustrative examples of such an antibody fragment are single chain variable fragments (scFv), Fv fragments, single domain antibodies, such as e.g. VHH (camelid) antibodies, di-scFvs, fragment antigen binding regions (Fab), F(ab')₂ fragments, Fab' fragments, diabodies or domain antibodies, to name only a few (Holt LJ1, Herring C, Jespers LS, Woolven BP, Tomlinson IM. Domain antibodies: proteins for therapy. Trends Biotechnol. 2003 Nov; 21(11):484-90).

For example, a blocking-reagent used in the present invention can be an antibody or a divalent antibody fragment such as an (Fab)₂'-fragment or a divalent single-chain Fv fragment. Therefore, a blocking-reagent used in the present invention can be an antibody or antibody fragment, which has an antibody format as depicted in Fig. 5 or as described in International patent application WO2013/092001. Alternatively, the blocking-reagent might also be a bivalent proteinaceous artificial binding molecule such as a lipocalin mutein that is also known as "duocalin".

In other embodiments, a blocking-reagent used in the present invention may only have a single binding site, i.e., may be monovalent. Examples of monovalent blocking-reagents include, but are not limited to, a monovalent antibody fragment, a proteinaceous binding molecule with antibody-like binding properties. Examples of monovalent antibody fragments include, but are not limited to a Fab fragment, a Fv fragment, and a single-chain Fv fragment (scFv). Therefore, a blocking-reagent used in the present invention may also be an antibody or antibody fragment, which has an antibody format as depicted in Fig. 6.

In some embodiments, antibody derived blocking-reagents that are used in the present invention may comprise an attenuated Fc-part. An Fc-part is, for example, attenuated, when such an antibody molecule is not able to bind via the CH2 or the CH3 domain to Fc receptors anymore, or binds less efficiently to them than a parent antibody. Examples of mutations that can be introduced into the CH2 or CH3 domain to achieve such Fc attenuation are described in International patent application WO2013/092001 (cf. for example, Figures 1 N, O of WO 2013/092001). In other embodiments, antibody derived blocking-reagents used in the present invention may comprise no Fc part at all (see, for example blocking antibody molecules such as blinatumomab).

A blocking-reagent used in the present invention can also be a proteinaceous binding molecule with antibody-like binding properties. Illustrative examples of proteinaceous binding molecules with antibody-like binding properties that can be used as blocking-reagent include, but are not limited to, an aptamer, a mutein based on a polypeptide of the lipocalin family, a glubody, a protein based on the ankyrin scaffold, a protein based on the crystalline scaffold, an adnectin, an avimer, a EGF-like domain, a Kringle-domain, a fibronectin type I domain, a fibronectin type II domain, a fibronectin type III domain, a PAN domain, a G1a domain, a SRCR domain, a Kunitz/Bovine pancreatic trypsin Inhibitor domain, tendamistat, a Kazal-type serine protease inhibitor domain, a Trefoil (P-type) domain, a von Willebrand factor type C domain, an Anaphylatoxin-like domain, a CUB domain, a thyroglobulin type I repeat, LDL-receptor class A domain, a Sushi domain, a Link domain, a Thrombospondin type I domain, an immunoglobulin domain or a an immunoglobulin-like domain (for example, domain antibodies or camel heavy chain antibodies), a C-type lectin domain, a MAM domain, a von Willebrand factor type A domain, a Somatomedin B domain, a WAP-type four disulfide core domain, a F5/8 type C domain, a Hemopexin domain, an SH2 domain, an SH3 domain, a Laminin-type EGF-like domain, a C2 domain, "Kappabodies" (III CR1, Gonzales JN, Houtz EK, Ludwig JR, Melcher ED, Hale JE, Pourmand R, Keivens VM, Myers L, Beidler K, Stuart P, Cheng S, Radhakrishnan R. Design and construction of a hybrid immunoglobulin domain with properties of both heavy and light chain variable regions. Protein Eng. 1997 Aug;10(8):949-57) "Minibodies" (Martin F1, Toniatti C, Salvati AL, Venturini S, Ciliberto G, Cortese R, Sollazzo M. The affinity-selection of a minibody polypeptide inhibitor of human interleukin-6. EMBO J. 1994 Nov 15;13(22):5303-9), "Janusins" (Traunecker A, Lanzavecchia A, Karjalainen K. Bispecific single chain molecules (Janusins) target cytotoxic lymphocytes on HIV infected cells. EMBO J. 1991 Dec;10(12):3655-9 and Traunecker A, Lanzavecchia A, Karjalainen K. Janusin: new molecular design for bispecific reagents. Int J Cancer Suppl. 1992;7:51-2), a nanobody, an adnectin, a tetranectin, a microbody, an affilin, an affibody or an ankyrin, a crystallin, a knottin, ubiquitin, a zinc-finger protein, an autofluorescent protein, an ankyrin or ankyrin repeat protein or a leucine-rich repeat protein, an avimer (Silverman J1, Liu Q, Bakker A, To W, Duguay A, Alba BM, Smith R, Rivas A, Li P, Le H, Whitehorn E, Moore KW, Swimmer C, Perlroth V, Vogt M, Kolkman J, Stemmer WP. Multivalent avimer proteins evolved by exon shuffling of a family of human receptor domains. Nat Biotechnol. 2005 Dec;23(12):1556-61. Epub 2005 Nov 20); as well as multivalent avimer proteins evolved by exon shuffling of a family of human receptor domains as also described in Silverman et al. (2005) cited above). In some embodiments, the blocking-reagent used in the present invention is a proteinaceous binding molecule with antibody-like binding properties, which is selected from the group of an aptamer, a mutein based on a polypeptide of the lipocalin family, a glubody, a protein based on the ankyrin scaffold, a protein based on the crystalline scaffold, an adnectin, and an avimer.

Alternatively, a blocking-reagent used in the present invention can also be a non-proteinaceous aptamer. Such an aptamer is an oligonucleic acid that binds to a specific target molecule. These aptamers are usually created by selecting them from a large random sequence pool, but natural aptamers also exist. More specifically, aptamers can be classified as: DNA or RNA aptamers. They consist of (usually short) strands of oligonucleotides. Therefore, a proteinaceous aptamer as described above may also include an oligonucleotide portion in addition to a protein portion.

As described above, one way to provide a blocking-reagent that binds to a cell adhesion molecule and/or cytokine is to provide an antibody molecule binding one of these molecules of interest. Therefore, the blocking-reagent can, for example, be an anti-CD11a antibody, an anti-CD11b antibody, an anti-CD11c antibody, an anti-CD275 antibody, an anti-CD54 antibody, an anti-CD102 antibody, an anti-CD86 antibody, an anti-LFA1 antibody, an anti-CD2 antibody, anti-TNFalpha antibody or an anti-CD18 antibody.

In preferred embodiments, the antibody may be an anti-CD275 antibody, an anti-CD54 antibody, an anti-CD102 antibody, an anti-TNFalpha antibody, a combination of an anti-CD54 and an anti-CD102 antibody, an anti-CD2 antibody and/or an anti-CD18 antibody, most preferably the antibody is an anti-CD18 antibody. In particular preferred embodiments, the blocking-reagent may be an anti-CD18 antibody and/or anti-CD2 antibody and/or an anti-CD54 antibody.

The person skilled in the art understands that also combinations of different blocking-reagents can be used in the present invention. For example, it is possible to use a combination of an anti-CD18 antibody, or an anti-CD2 antibody with an anti-CD54 antibody. Also combinations of these blocking-reagents with any of the other blocking-reagents used in the present invention are possible. In this respect, also e.g. bispecific (or trispecific) antibody molecule formats as described e.g. in Fig. 5 or as described in International patent application WO2013/092001 can be utilized.

Among the different blocking-reagents that can be used in the present invention, a blocking-reagent binding to the CD18 performed particularly well in the Examples described herein. Without being bound to theory it is suggested that the anti-CD18 blocking-reagent is particularly effective in reducing unspecific T cell activation, because it very effectively blocks/reduces cell adhesion between T cells and bystander cells.

Under physiological conditions, CD18 (Integrin beta-2) is usually present in a complex with other proteins. This is because CD18 is an integrin, which are integral cell-surface proteins composed of an alpha chain and a beta chain. A given beta chain such as CD18 can combine with multiple partners resulting in different integrins. Furthermore, integrins are also bound by various ligands. Ligand binding can for example regulate the shape, orientation, and movement of cells or activate intracellular signaling pathways.

Accordingly, CD18 can be the beta subunit of four different structures:
(1) LFA-1 (CD18 paired with CD11a), which is expressed on all leucocytes; ligands include e.g. ICAM-1, ICAM-2, ICAM-3, ICAM-4, ICAM-5 and JAM-1 (Tan SM. The leucocyte β2 (CD18) integrins: the structure, functional regulation and signalling properties. Biosci Rep. 2012 Jun; 32(3):241-69);
(2) Macrophage-1 antigen (CD18 paired with CD11b), expressed on monocytes, macrophages, NK cells, neutrophils and some T cells; exemplary ligands are ICAM-1, ICAM-2, ICAM-3, ICAM-4, JAM-3, Factor X, heparin, neutrophil inhibitory factor, MBP, high molecular mass kininogen, microbial saccharides, plasminogen, fibronectin, laminin, collagen II and VI, collagen I, tissue growth factor, RAGE, cysteine-rich 61, connective denatured proteins uPAR and more (Tan (2012 cited above);
(3) Integrin alphaXbeta2 (CD18 paired with CD11c), which is expressed on monocytes, macrophages dendritic cells and NK cells; ligands include many of (2) including e.g. fibrinogen, ICAM-1, ICAM-4, LPS, collagen, heparin, denatured proteins (Tan (2012 cited above) and
(4) Integrin alphaDbeta2 (CD18 paired with CD11d) expressed on macrophages and eosinophils; ligands are e.g. ICAM-3 and VCAM-1 (Tan (2012 cited above). The described integrins are involved in virtually every aspect of leukocyte function, including the immune response, adhesion to and transmigration through the endothelium, phagocytosis of pathogens, and leukocyte activation (Edward F. Plow, Thomas A. Haas, Li Zhang, Joseph Loftus and Jeffrey W. Smith Ligand Binding to Integrins. July 21, 2000. The Journal of Biological Chemistry, 275, 21785-21788).

Due to the combinatorial structure of integrins, a blocking-reagent used herein can be directed to CD18 itself or, alternatively, to any one of its combinatory partners. This means, that for example, also anti-LFA1 antibodies, anti-CD11b antibodies, anti-CD11a antibodies or anti-CD11d antibodies may be used in the present invention, alone or in combination with other blocking-reagents described herein. One example of such an antibody is efalizumab, which binds to CD11a.

Alternatively, a blocking-reagent may also target any of the ligands, which bind to CD18 and/or its combinatory partner. Without wishing to be bound to theory it is believed that the ligand binding pocket consists of portions of both, the α and the β subunits (Edward et al. (2000) cited above). By binding to a ligand that binds to CD18 and its combinatory partner, a blocking-reagent may as well interfere with CD18 functions. Especially such a blocking-reagent may interfere with CD18/integrin mediated signaling. Particularly, an anti-ICAM-1 antibody (CD54) and a combination of an anti-ICAM-1 and anti-ICAM-2 antibody has been shown to be particularly useful in the present invention. Other exemplary blocking-reagents that can affect CD18 functions via binding to a CD18/integrin ligand can include R6.5 (BIRR-1, enlimomab) a murine IgG2a mAb to the human ICAM-1 or BI-505 which is a fully human antibody binding to the adhesion protein ICAM-1 (CD54).

However, in preferred embodiments of the present invention blocking-reagents that bind specifically (or exclusively) to CD18 are used.

By "a blocking reagent that binds specifically to CD18" is meant a blocking-reagent that specifically binds to an epitope of CD18 (i.e. an epitope that is solely formed by CD18 residues) but not to an epitope that is formed by one of its combinatory partners CD11a, CD11b, CD11c and CD11d. Such an epitope can either be a linear epitope or a conformational epitope. For the avoidance of doubt, it is mentioned here, that the term "epitope" has traditionally been used to refer to the region of an antigen to which an antibody would bind via its antigen binding site. However, since artificial binding molecules with antibody-like properties are now readily available, the term "epitope" refers also to the region to which, for example, such artificial binding molecules, as for example, an anti-CD-18 lipocalin mutein (Anticalin®) would bind with their binding site. In this context one illustrative example of a CD18 blocking-reagent that bind to an epitope that is solely formed by CD18 residues include, but are not limited to the antibody b2/TS 1/18 (Sanchez-Madrid F., Nagy J.A., Robbins E., Simon P., Springer T. (1983) The lymphocyte function-associated antigen (LFA-1), the C3bi complement receptor (OKM1/Mac-1), and the p150,95 molecule. J. Exp. Med. Vol. 158, p. 1785-1803) that has been used in the example section of the present application. Other illustrative examples of such CD18 binding blocking-reagents are the antibodies Erlizumab (also known as rhuMab CD18) and Rovelizumab (LeukArrest® or Hu23F2G). As described for example in the International patent application WO 92/22653, Erlizumab is a humanized derivate of the monoclonal murine antibody H52 that, as described in US patent 5,888,508) specifically binds to CD18. Similarly, as described in US patent 5,854,070 or European patent application 0 578 51, the antibody Rovelizumab is a humanized version of the monoclonal murine antibody 60.3 which also specifically binds CD18 (see Beatty et al. J. Definition of a common leukocyte cell-surface antigen (Lp95-150) associated with diverse cell-mediated immune functions. J. Immunol. 131: 2913-2918, 1983). Thus, from these examples, it is also evident that, if an antibody molecule is used as blocking-reagent, the antibody molecule can be a polyclonal or monoclonal antibody obtained by immunization or a recombinant antibody such as a chimeric antibody, a humanized antibody or an antibody fragment obtained by evolutionary methods such as phage display.

In addition to the above, encompassed in the term "a blocking reagent that binds specifically to CD18" are also blocking-reagents that bind specifically bind to an epitope that is formed by residues of both CD18 and one of its combinatory partners CD11a, CD11b, CD11c and CD11d. Thus, such a blocking-reagent binds to a complex that is formed by CD18 and CD11 (a, b, c or d), also referred to as the CD18/CD11 complex. Such an epitope to which both partners of the complex contribute is usually referred to as a conformational epitope. Again in this case, such a "CD18 blocking reagent" as used herein does not bind to CD11 (a, b, c or d) alone, meaning the CD18 blocking reagent does not bind to an epitope formed by residues of CD11a, CD11b, CD11 c and CD11 d alone.

A blocking-reagent that binds to CD18 has the advantage that e.g. anti-CD-18 antibodies such as antagonistic CD18 antibody molecules have already been developed in the 1990ies by different companies (see, for example, the above-mentioned antibody molecules Erlizumab (also known as rhuMab CD18) developed by Genentech/Roche and Rovelizumab (LeukArrest® or Hu23F2G) developed by Icos Coorp).

Therefore, such antibodies are already available to be used as blocking-reagent in the present invention. These antibodies were, for example, developed to prevent the migration of leucocytes to sites of potential inflammation (Ulbrich H, Eriksson EE, Lindbom L. Leukocyte and endothelial cell adhesion molecules as targets for therapeutic interventions in inflammatory disease. Trends Pharmacol Sci 2003; 24:640-647). Importantly, these different anti-CD18 blocking-reagents have also already been applied to humans in different clinical studies and were shown to be safe to use, meaning they constitute readily available candidate molecules for *in vivo* co-administration with bispecific antibodies such as blinatumomab or CAR T cells.

In more detail, it is noted here that in one clinical trial that the humanized monoclonal anti-CD18 antibody molecule rovelizumab (that is also known as Hu23F2G) that binds to and blocks the functions of the CD11/CD18 integrin was analyzed in patients after acute myocardial infarction, who underwent percutaneous transluminal angioplasty or patients with multiple sclerosis. In both studies, administration of Hu23F2G was well tolerated (Rusnak JM, Kopecky SL, Clements IP, Gibbons RJ, Holland AE, Peterman HS, Martin JS, Saoud JB, Feldman RL, Breisblatt WM, Simons M, Gessler CJ Jr, Yu AS. An anti-CD11/CD18 monoclonal antibody in patients with acute myocardial infarction having percutaneous transluminal coronary angioplasty (the FESTIVAL study). Am J Cardiol 2001; 88:482-487 and Bowen JD, Petersdorf SH, Richards TL, Maravilla KR, Dale DC, Price TH, St John TP, Yu AS. Phase I study of a humanized anti-CD11/CD18 monoclonal antibody in multiple sclerosis. Clin Pharmacol Ther 1998; 64:339-346). Furthermore, Hu23F2G therapy did not show adverse events, including infections (Rusnak et al., 2001 cited above). In addition, it was found that Hu23F2G was also tolerated at doses that achieved high degrees of leucocyte CD11/CD18 saturation with *in vivo* inhibition of leucocyte migration (Bowen et al., (1998) cited above).

Also the CD18 binding humanized monoclonal antibody F(ab')₂ fragment rhuMab CD18 (also known as erlizumab) has been investigated in clinical trials (Rhee P, Morris J, Durham R, Hauser C, Cipolle M, Wilson R, Luchette F, McSwain N, Miller R. Recombinant humanized monoclonal antibody against CD18 (rhuMAb CD18) in traumatic hemorrhagic shock: results of a phase II clinical trial. Traumatic Shock Group. J Trauma 2000; 49:611-619). It was found that the administration of rhuMab CD18 resulted in a dose-dependent saturation of CD18 expression on neutrophils. For example, the 2 mg/kg dosage resulted in greater than 90% neutrophil CD18 receptor saturation for approximately 48 hours. In the 2mg/kg group the mortality was 0%. Also rhuMab CD18 was well tolerated and effective in neutrophil CD18 receptor saturation (Baran KW, Nguyen M, McKendall GR, Lambrew CT, Dykstra G, Palmeri ST, Gibbons RJ, Borzak S, Sobel BE, Gourlay SG, Rundle AC, Gibson CM, Barron HV; Limitation of Myocardial Infarction Following Thrombolysis in Acute Myocardial Infarction (LIMIT AMI) Study Group. Double-blind, randomized trial of an anti-CD18 antibody in conjunction with recombinant tissue plasminogen activator for acute myocardial infarction: limitation of myocardial infarction following thrombolysis in acute myocardial infarction (LIMIT AMI) study. Circulation 2001; 104:2778-2783 and Rhee P, Morris J, Durham R, Hauser C, Cipolle M, Wilson R, Luchette F, McSwain N, Miller R. Recombinant humanized monoclonal antibody against CD18 (rhuMAb CD18) in traumatic hemorrhagic shock: results of a phase II clinical trial. Traumatic Shock Group. J Trauma 2000; 49:611-619)

While the development of these anti-CD18 antibodies has been halted because clinical trials with patients suffering from myocardial infarction (Adams and Weiner (2005) cited above; Topp et al., (2011) cited above; Kroesen et al., (1994) cited above), hemorrhagic shock (Rhee et al., (2000) cited above), or multiple sclerosis were unsuccessful (Bowen et al., (1998) cited above), these studies have demonstrated that blocking concentrations of such antibody molecules can be safely achieved in human patients. Furthermore, for the purposes of the present invention, an anti-CD18 blocking-reagent binding to CD18 does not need to have such a therapeutic effect. The purpose of an anti-CD18 blocking-reagent in accordance with the present invention is only decreasing/interfering with the interaction between bystander cells and T cells. Thus, a mere binding of an anti-CD18 antibody to CD18 is sufficient for the purposes of the present invention.

Other exemplary blocking-reagents apart from Hu23F2G and erlizumab are the monoclonal antibody 6.7, which reacts with human and non-human primate (rhesus or cynomologus) CD18 (David V, Leca G, Corvaia N, Le Deist F, Boumsell L, Bensussan A. (1991) Proliferation of resting lymphocytes is induced by triggering T cells through an epitope common to the three CD18/CD11 leukocyte adhesion molecules. Cell Immunol. 136(2):519-24), the anti-CD18 monoclonal antibody MHM23, which detects CD18 from human samples (Hildreth JE, Gotch FM, Hildreth PD, McMichael AJ. (1983) A human lymphocyte-associated antigen involved in cell-mediated lympholysis. Eur J Immunol. 13(3):202-8), the MAS191 c antibody (Vermot Desroches C, Rigal D, Andréoni C. (1991) Regulation and functional involvement of distinct determinants of leucocyte function-associated antigen 1 (LFA-1) in T-cell activation in vitro. Scand J Immunol. 33(3):277-86), the IOT 18 antibody (Ricevuti G, Mazzone A, Pasotti D, Fossati G, Mazzucchelli I, Notario A (1993) The role of integrins in granulocyte dysfunction in myelodysplastic syndrome. Leuk Res. 17(7):609-19), the b2/TS 1/18 antibody (Sanchez-Madrid F., Nagy J.A., Robbins E., Simon P., Springer T. (1983) The lymphocyte function-associated antigen (LFA-1), the C3bi complement receptor (OKM1/Mac-1), and the p150,95 molecule. J. Exp. Med. Vol. 158, p. 1785-1803), the murine antibody 60.3 (Beatty PG, Ledbetter JA, Martin PJ, Price TH, Hansen JA (1983) Definition of a common leukocyte cell-surface antigen (Lp95-150) associated with diverse cell-mediated immune functions. J Immunol. 131 (6):2913-8 and Vedder N.B., Winn R.K., Rice C.L., Chi E.Y., Arfors K.E. Harlan J.M. (1990) Inhibition of leukocyte adherence by anti-CD18 monoclonal antibody attenuates reperfusion injury in the rabbit ear. Natl. Acad. Sci. Vol. 87, pp. 2643-2646), the antibody KIM127 (ATCC No: CRL-2838), the antibody IB4 (ATCC No: HB-10164) and humanized versions thereof (see International patent application WO 01/70260), the above-mentioned murine antibody H52 (ATCC No: HB10160), the latter distributed by the American Type culture collection (ATCC, Manassas, USA) or an antibody produced by ATCC TIB-218.

The above described blocking-reagents are effective in reducing unspecific T cell activation of bispecific antibody molecule therapy and CAR T cell therapy. In general a "therapy" seeks remediation of a health problem, usually following a diagnosis. In the medical field, this term is synonymous with treatment of a disease or disorder. Therefore, in this context, a therapy also includes the administration of bispecific antibody molecules or CAR modified T cells. Likewise, a "therapeutic effect" relieves to some extent one or more of the symptoms of the abnormal condition.

A bispecific antibody molecule used in therapy in accordance with the present invention comprises two binding sites wherein the first binding site binds to an antigen associated with a target cell and wherein the second binding site binds to a TCR/CD3 complex, on an effector cell.

The first binding site of the bispecific antibody molecule can for example bind a tumor associated antigen expressed by a target cell as described above. Alternatively, such a bispecific antibody molecule can bind an antigen associated with an autoimmune disease, which can also be expressed by a target cell as described above.

The second binding site of the bispecific antibody molecule can bind a TCR or a CD3-molecule within the TCR/CD3 complex. More particularly, the second binding site of the bispecific antibody molecule can bind to CD3. To be able to bind to CD3, the second binding site of a bispecific antibody molecule used in therapy in accordance with the present invention can comprise a binding site of an anti-CD3 antibody. For example, the second binding site can comprise a binding site of the UCHT1 antibody, which has a sequence identity of at least 80%, or at least 85 %, or at least 90 %, or at least 95 %, or at least 98 %, or at least 99 % or 100 % to SEQ ID NO. 1 (the sequence of the light chain of the variable domain of UCHT-1) and/or the bispecific antibody molecule comprises a binding site of the UCHT-1 antibody, which has a sequence identity of at least 80%, or at least 85 %, or at least 90 %, or at least 95 %, or at least 98 %, or at least 99 % or 100 % to SEQ ID NO. 2 (the sequence of the heavy chain of the variable domain of UCHT-1). In other embodiments, the bispecific antibody molecule comprises a binding site of the OKT3 antibody, which has a sequence identity of at least 80%, or at least 85 %, or at least 90 %, or at least 95 %, or at least 98 %, or at least 99 % or 100 % to SEQ ID NO. 3 (the sequence of the light chain of the variable domain of OKT3) and/or the bispecific antibody molecule comprises a binding site of the OKT3 antibody, which has a sequence identity of at least 80%, or at least 85 %, or at least 90 %, or at least 95 %, or at least 98 %, or at least 99 % or 100 % to SEQ ID NO. 4 (the sequence of the heavy chain of the variable domain of OKT3). Other examples of CD3 binding antibody molecules that can be used in the present invention include the antibody molecules described in European Patent 2 155 783 B1 or European Patent EP 2 155 788 B1 that are capable of binding to an epitope of human and *Callithrix jacchus, Saguinus oedipus* or *Saimiri sciureus* CD3ε chain.

In general, a bispecific antibody molecule used in therapy in accordance with the present invention can be present in different antibody formats, which are known to the skilled artesian and summarized in (Roeland et al., (2014) cited above). Illustrative examples include but are not limited to a monoclonal antibody (mAb), a triomab antibody, a F(ab')₂ antibody, a scFv antibody, a TaFv antibody, a bsDb antibody, a DART antibody, a heavy chain only antibody, or a bsVHH antibody (as depicted in Fig. 5). Thus, the bispecific antibody molecule can also be present in any antibody format as depicted in Fig. 5 or as described in WO2013092001.

Different bispecific antibody molecules have already been used in clinical settings. Examples of such bispecific antibody molecules that can also be used in therapy in accordance with the present invention include catumaxomab (removab, anti-EpCAM x anti-CD3), ertumaxomab (anti-HER2 x anti-CD3), SHR-1 (anti-CD3 x anti-CD19), blinatumomab, CBA-CEACD3 (CD3 x CEA), BIS-1 (anti-EGP-2 x anti-CD3), MT-110 (anti-EpCAM x anti-CD3), EGFRBi (anti-CD3 x anti-EGFR BiAb), CD20Bi (anti-CD3 x anti-CD20 BiAb), MGD006 (anti-CD123 x anti-CD3), FBTA05 (anti-CD20 x anti-CD3), MGD007 (anti-gpA33 x anti-CD3), MOR209/ES414 (anti-PSMA x anti-CD3), BAY2010112 (anti-PSMA x anti-CD3), triomab antibodies such as anti-CD3 x anti-EpCAM triomab and EGFRXCD3 bsFab₂ (Jung et al. Int J Cancer Local immunotherapy of glioma patients with a combination of 2 bispecific antibody fragments and resting autologous lymphocytes: evidence for in situ t-cell activation and therapeutic efficacy Jan 15;91(2):225-30, 2001). In one particular example, the bispecific antibody molecule binds CD3 and CD19. Examples of such bispecific CD3 x CD19 antibody molecules include those single-chain antibody molecules that are described in International Patent Applications WO 99/54440 and WO 2004/106381. A particularly preferred single chain antibody molecule of those described in WO 99/54440 and WO 2004/106381 that is used in the present invention is the antibody molecule blinatumomab (for a current review of the properties of blinatumomab see Portell et al, Clinical and pharmacologic aspects of blinatumomab in the treatment of B-cell acute lymphoblastic leukemia, Clinical Pharmacology: Advances and Applications 2013:5 (Suppl 1) 5-11) or an antibody molecule that carries the six CDR regions of blinatumomab. In addition, antibody molecules the variable domains of which have at least 70 %, 75 %, 80%, 85 %, 90 %, 92 %, 94 %, 95 %, 96 %, 97 %, 98 % or 99 % sequence identity with blinatumomab (SEQ ID NO: 5) are also preferred in the present invention. It is noted here that the term "sequence identity" as used in the present invention means the percentage of pair-wise identical residues - following (homology) alignment of a sequence of a polypeptide of the invention with a sequence in question - with respect to the number of residues in the longer of these two sequences. Identity is measured by dividing the number of identical residues by the total number of residues and multiplying the product by 100.

In addition to the co-administration of blocking reagents with bispecific antibody molecules as described herein, the present invention also relates to the use of blocking-reagents in CAR modified T cell therapy.

A CAR modified T cell in general comprises engineered receptors, which graft an arbitrary specificity onto an immune effector cell. For therapy, typically first native T cells are removed from a patient and modified so that they express chimeric receptors specific to an antigen associated with a target cell (autologous T cells). These chimeric antigen receptors (CAR) combine an antibody molecule comprising a binding site that binds to an antigen associated with a target cell (binding site) which is connected with or fused to the signal activating machinery of a T cell as the TCR/CD3 signaling domain (signaling portion). The CAR modified T cells can then recognize and kill target cells, when they are reintroduced into the patient.

The binding site of the CAR can, in an analogous manner to the bispecific antibody molecule, bind to a tumor associated antigen and/or an antigen associated with an autoimmune disease, which is expressed on a target cell as described above. The tumor associated antigen to which the binding site of the CAR binds, may for example, also be selected from the group consisting of CD19, CD20, CD30, CD33, CD138, Lewis Y, EGFR and Ig kappa light chain (κ).

In some embodiments, the CAR comprises an antibody molecule, which is single-specific or bispecific. These single-specific or bispecific antibody molecules can be present in an antibody format as depicted in Figures 5 or 6. In other embodiments, the CAR comprises an antibody molecule, which comprises a scFv. Such a scFv can be derived from e.g. a TAA-specific monoclonal antibody.

In addition to the antibody molecule comprising a binding site that binds to an antigen associated with a target cell, the modified CAR of the CAR modified T cells also comprises a TCR/CD3 signaling domain, which is fused to the antibody molecule of the CAR. This fusion can also be a fusion via a hinge region. Suitable hinge regions are, for example, IgG-CD4, IgG-CD28, CD28, CD8-CD8, IgG1-CD4, or CD8-CD28. Often the hinge region also comprises a transmembrane domain.

The TCR/CD3 signaling domain is located mostly within the cell membrane and intracellular in T cells, i.e. T lymphocytes. The whole T cell receptor generally has two separate peptide chains, typically T cell receptor alpha and beta (TCRα and TCRβ) chains, on some T cells T cell receptor gamma and delta (TCRγ and TCRδ). The other proteins in the complex are the CD3 proteins: CD3εγ and CD3εδ heterodimers and, most important, a CD3ζ homodimer, which has a total of six ITAM motifs. The ITAM motifs on the CD3ζ can be phosphorylated by Lck and in turn recruit ZAP-70. Lck and/or ZAP-70 can also phosphorylate the tyrosines on many other molecules, not least CD28, LAT and SLP-76, which allows the aggregation of signaling complexes around these proteins.

Therefore, in some embodiments, the TCR/CD3 signaling domain comprises a CD3ζ domain. However, in other embodiments, the TCR/CD3 signaling domain comprises a CD3ζ domain and one co-stimulatory domain. This co-stimulatory domain can be selected from the group consisting of 4-1 BB or CD28. Alternatively, the TCR/CD3 signaling domain can also comprise a CD3ζ domain and two co-stimulatory domains. In this case, the co-stimulatory domains can be selected from the group consisting of 4-1 BB, CD28, CD27, OX40 or ICOS. More specifically, the TCR/CD3 signaling domain can for example contain a 4-1BB-CD3ζ, CD28-CD3ζ, CD28-4-1BB-CD3ζ, CD3ζ, CD137- CD3ζ, anti-Lewis Y-CD28-CD3ζ.

Also different CAR modified T cells have already been used in the clinic (see also Maus et al., (2014) cited above) and can therefore be subject of the present invention. Exemplary CAR modified T cells that can be administered for therapy as described herein include but are not limited to CD19:4-1BB:CD3 modified T cells (NCT01029366), aCD19z cells (NCT01493453), anti-LeY-scFv-CD28-ζ vector modified T cells (NCT01716364), CD19-CAR T cells (NCT02028455), T1 E28z T cells (NCT01818323), GD2-CAR T cells (NCT01953900), CD19.CAR-CD28Z T cells (NCT02050347), anti-CD19-CAR vector-transduced T cells (NCT02081937), 19-28z T cells (NCT01840566), CART-19 T cells (NCT01747486), CM-CS1 T cells (targets NKG2D; NCT02203825), autologous HER2-specific T cells (NCT00902044), CART-EGFRvIII T cells (NCT02209376), third generation CAR-T cells (NCT02186860), anti-CD19-chimeric-antigenreceptor-transduced T cells (NCT01087294), CART30 T cells (NCT02259556), anti-GD2-CAR T cells (GD2-CAR.OX40.28.z.ICD9; NCT02107963), iC9-GD2 T Cells (NCT01822652), genetically modified HER.CAR CMV-specific T cells (NCT01109095), CART33 T cells (NCT01864902), CART-meso T cells (NCT02159716), CAR.CD30 T cells (NCT01316146), CD19 specific CAR T cells (3^{rd} generation; NCT01853631), CART-138 T cells (NCT01886976), kappa CD28 T cells (NCT00881920), anti-EGFRvIII CAR T cells (NCT01454596), autologous anti-CD19CAR-4-1BB-CD3zeta-EGFRt-expressing T lymphocytes (NCT01865617), anti CD123-CAR/CD28-costimulatory T cells (NCT02159495), anti-CD19-CAR T cells (NCT01593696), CART-19 T cells (NCT02030834), CD19CAR T cells (NCT01430390), anti-CD19 CAR T cells (NCT02247609), CART-19 T cells (NCT02030847), CD19.CAR T cells (Cruz CR1, Micklethwaite KP, Savoldo B, Ramos CA, Lam S, Ku S, Diouf O, Liu E, Barrett AJ, Ito S, Shpall EJ, Krance RA, Kamble RT, Carrum G, Hosing CM, Gee AP, Mei Z, Grilley BJ, Heslop HE, Rooney CM, Brenner MK, Bollard CM, Dotti G. Infusion of donor-derived CD19-redirected virus-specific T cells for B-cell malignancies relapsed after allogeneic stem cell transplant: a phase 1 study. Blood. 2013 Oct 24;122(17):2965-73), anti-Lewis-Y (anti-LeY) CAR modified T cells (Maus et al., (2014) cited herein), 19-28z T cells (Brentjens et al., (2013) cited above) and/or T cells modified with CARs based on the humanized monoclonal antibody trastuzumab and the CD28, 4-1 BB and CD3ζ (Morgan et al., (2010) cited herein).

A blocking-reagent used in the present invention as well as a bispecific antibody molecule and/or a CAR modified T cell used in therapy in accordance with the present invention can all be administered to a subject. The term "administration" means administering of a therapeutically or diagnostically effective dose of the blocking-reagent as well as the bispecific antibody molecule and/or CAR modified T cell to a subject. The term "administering" also relates to a method of incorporating a compound into cells or tissues of an organism. Different routes of administration are possible and are described below. In some embodiments, the blocking-reagent and the bispecific antibody molecule are administered simultaneously or sequentially. Thus, the blocking-reagent can be administered before the bispecific antibody molecule is administered. Alternatively, the blocking-reagent can also be administered after the bispecific antibody molecule has been administered. Similarly, the blocking-reagent and the CAR modified T cell can be administered simultaneously or sequentially. Again, the blocking-reagent can be administered before the CAR modified T cell is administered. In contrast, the blocking-reagent can also be administered after the CAR modified T cell has been administered. Notably, the term "therapeutic effect" refers to the inhibition or activation of factors causing or contributing to the abnormal condition.

The blocking-reagent used in the present invention as well as the bispecific antibody molecule or CAR modified T cell can be administered via different ways such as any parenteral or non-parenteral (enteral or topical) route that is therapeutically effective for (preferably proteinaceous) drugs. Parenteral application methods include, for example, subcutaneous, intramuscular, intracerebral, intracerebroventricular, intrathecal, intranasal, intra-atrial, intraperitoneal or intravenous injection and infusion techniques, e.g. in the form of injection solutions, infusion solutions or tinctures. Non-parenteral delivery modes are, for instance, enteral delivery modes such as oral delivery, e.g. in the form of pills, tablets, capsules, solutions or suspensions, or rectally, e.g. in the form of suppositories. Topical application routes include epicutaneous or inhalational applications. An overview about pulmonary drug delivery, i.e. either via inhalation of aerosols (which can also be used in intranasal administration) or intracheal instillation is given by Patton et al. (2004) for example (J.S. Patton et al. The lungs as a portal of entry for systemic drug delivery. Proc. Amer. Thoracic Soc. 2004 Vol. 1 pages 338-344). In general, blocking-reagents used in the present invention as well as bispecific antibody molecules or CAR modified T cells can be administered in formulations containing conventional non-toxic pharmaceutically acceptable excipients or carriers, additives and vehicles as desired and described below.

In some embodiments, the blocking-reagent used in the present invention is administered in the same way and in the same injection/infusion solution as the bispecific antibody molecule or the CAR modified T cell. Alternatively, the blocking-reagent can be applied in a different way e.g. intraperitoneal, while the bispecific antibody molecule or the CAR modified T cell can be applied in another way e.g. intravenously.

Blocking-reagents used in the present invention can also be used in co-treatment with T cell engaging therapies. This co-treatment includes administration of a blocking-reagent used in the present invention, preferably in the form of a medicament, to a subject suffering from a condition receiving T cell engaging therapy. Similarly included is the administration of a blocking-reagent, preferably in the form of a drug/medicament, to a subject receiving T cell engaging therapy for the purpose of reducing side-effects.

As described above, bispecific antibody molecules and/or CAR modified T cells can be used in therapy. This therapy can be any therapy which is based on the engagement of T cells. Furthermore, any therapy directed at specific target cell associated antigens, involving engagement of T cells is meant by this term. For example, the therapy can be a therapy for treating a proliferatory disease or an autoimmune disease.

Examples of a proliferatory disease include hemopoetic malignancies, such as acute and chronic myeloic and lymphatic leukemias, as well as lymphomas, solid tumors such as tumors of the gastrointestinal tract, lung, kidney, prostate, breast, brain, ovary, uterus, mesenchymal tumors and melanoma. To analyze the effect of a blocking-reagents used in the present invention, for example, in cancer therapy, outcome measures can be selected e.g. from pharmacokinetics, immunogenicity, and the potential to decrease the size of a cancer by e.g. MRI imaging as well as patient reported outcomes.

Illustrative examples of autoimmune diseases are Systemic lupus erythematosus (SLE), Goodpasture's syndrome, Sarcoidosis, Scleroderma, Rheumatoid arthritis, Dermatomyositis, Sjögren's Syndrome, Scleroderma, Dermatomyositis, Psoriasis, Vitiligo, Alopecia areata, Type 1 diabetes mellitus, Autoimmune pancreatitis, Hashimoto's thyroiditis, Addison's disease, Multiple sclerosis, Myasthenia gravis, Polyarteritis nodosa, Idiopathic thrombocytopenic purpura, Hemolytic anemia, Antiphospholipid antibody syndrome, Pernicious anemia, Gastrointestinal diseases, Celiac disease, Inflammatory bowel disease, Autoimmune hepatitis or Primary biliary cirrhosis. To analyze the effect of a blocking-reagents used in the present invention for example in autoimmune therapy, outcome measures can be selected e.g. from pharmacokinetics, immunogenicity, and the potential to decrease autoimmune reactivity.

A blocking-reagent used in the present invention can also be used to reduce side-effects of T cell engaging therapies, such as bispecific antibody molecule therapy or CAR modified T cell therapy as described above. Especially, these "side-effects" are negative side-effects, which are not beneficial to the patients. Such side-effects can include at least one of cytokine release syndrome, skin rash, hearing loss, uveitis, inflammatory colitis, tumor lysis syndrome, fever, chills, dyspnea, fatigue, tachycardia, hypertension, back pain, vomiting, seizures, encephalopathy, edema, aseptic meningitis, nausea or headache. Further side-effects include rigor, malaise, myalgias, arthalgia, anorexia, diarrhea, tachypnea, hypoexemia, hypotension, increased cardiac output, widened pulse pressure, azotemia, transaminitis, hyperbilirubinemia, and mental status changes such as confusion, delirium, word finding difficulty, hallucinations, tremor or seizures. The reduction of side-effects can be measured by comparing the side-effects present in the presence of a blocking-reagent used in the present invention and the side-effects present in the absence of a blocking-reagent used in the present invention upon administration of bispecific antibody molecules and/or CAR modified T cells.

Notably, the side-effect termed "cytokine release syndrome" is a common immediate complication occurring with the use T cell engaging therapies as described above. Severe cases are known as cytokine storms. The pathogenesis of a cytokine release syndrome presumably is that T cells are activated. It is thinkable that such an activation of T cells may also be partly caused by effector cells that are (unspecifically) activated (unspecific T cell activation). The cytokines released by the activated T cells then produce a type of systemic inflammatory response similar to that found in severe infection characterized by hypotension, pyrexia and rigors. The patient may also suffer from fever. Typically, in the clinic cytokine release syndromes are reduced by using low dosages of the therapeutic such as bispecific antibody molecules, slow infusion instead of rapid injections, additional intravenous administration of a histamine antagonist and/or a corticosteroid prior to starting therapy or during the therapy.

A blocking-reagent used in the present invention can also be used to increase the dosages of bispecific antibody molecules and/or CAR modified T cells in their respective therapies. This possible application of the present invention is particularly interesting with regard to the severe dose limitations, observed in clinical trials with different bispecific antibody molecules (Kroesen BJ, Buter J, Sleijfer DT et al. Phase I study of intravenously applied bispecific antibody in renal cell cancer patients receiving subcutaneous interleukin 2. Br J Cancer 1994; 70:652-661 and Tibben JG, Boerman OC, Massuger LF et al. Pharmacokinetics, biodistribution and biological effects of intravenously administered bispecific monoclonal antibody OC/TR F(ab')2 in ovarian carcinoma patients. Int J Cancer 1996; 66:477-483), which are due to systemic cytokine release. Thus, in some embodiments, the blocking-reagents used in the present invention are used in therapy so that the dosage of the administered bispecific antibody and/or CAR modified T cell is increased compared to the dosage used without the blocking-reagent.

A "dosage" of a blocking-reagent used in the present invention as well as the bispecific antibody molecule or CAR modified T cell applied in accordance with the present invention may vary within wide limits to achieve the desired preventive effect or therapeutic response. It will, for instance, depend on the affinity of a blocking-reagent for a chosen target as well as on the half-life of the complex between a blocking-reagent or antibody molecule and the ligand *in vivo.* Further, the optimal dosage will depend on the biodistribution of a blocking-reagent used in the present invention as well as the bispecific antibody molecule or CAR modified T cells, the mode of administration, the severity of the disease/disorder being treated as well as the medical condition of the patient. For example, when used in an ointment for topical applications, a high concentration of the blocking-reagent as well as the bispecific antibody molecule or CAR modified T cell can be used.

Any suitable dosage of the bispecific antibody molecule or CAR modified T cells can be used in the present invention. Exemplary dosages of CAR modified T cells may include or be more than about 1.46 x 10⁵ to about 1.60 x 10⁷ CAR cells/kg body weight of the patient, may include or be more than about 1.5 to about 3.0 x 10⁶ autologous T cells/kg body weight, may include or be more than about 0.4 to about 3.0 x 10⁷ cells/kg body weight, or may include or be more than 1x 10⁸/m² to 3.3x10⁹)/m² skin surface of the patient (Maus MV, Grupp SA, Porter DL, June CH. Antibody-modified T cells: CARs take the front seat for hematologic malignancies. Blood 2014; 123:2625-35). Exemplary dosages of bispecific antibody molecules may include or be more than about 0.0005 to about 0.006 mg/m²/day or may include or be more than about 10 to about 200 µg (Burges A, Wimberger P, Kümper C, Gorbounova V, Sommer H, Schmalfeldt B, Pfisterer J, Lichinitser M, Makhson A, Moiseyenko V, Lahr A, Schulze E, Jäger M, Ströhlein MA, Heiss MM, Gottwald T, Lindhofer H, Kimmig R. Effective relief of malignant ascites in patients with advanced ovarian cancer by a trifunctional anti-EpCAM x anti-CD3 antibody: a phase I/II study. Clin Cancer Res. 2007 Jul 1; 13(13):3899-905). The blocking-reagent can also be used in any suitable dosage. It is within knowledge of the person of average skill in the art to, for example, empirically determine a suitable dosage of the blocking reagent. In illustrative embodiments, the blocking reagent such as an anti-CD18 blocking-reagent can be used in a dosage of about 0.3 mg/kg body weight of the patient, of about 0.5 mg/kg body weight, of about 1 mg/kg body weight, of about 2 mg/kg body weight, or even a higher dosage.

As outlined above, due to side-effects observed in T cell engaging therapies, such therapy usually includes low-dosage applications and slow infusion protocols of the bispecific antibody molecules and/or CAR modified T cells. Therefore, a blocking-reagent used in the present invention may also be used to increase the speed of the application of administered bispecific antibody molecules and/or CAR modified T cells. For example, an administered bispecific antibody molecule and/or CAR modified T cell may be more rapidly injected when combined with the application of a blocking-reagent used in the present invention when compared to the speed used without a blocking-reagent. More importantly, the doses applicable without inducing major side effects may be substantially increased.

A blocking-reagent used in the present invention as well as bispecific antibody molecules and CAR modified T cells are applied to a subject. The term "subject" can also mean an individual/patient receiving a treatment of bispecific antibody molecule therapy or CAR modified T cell therapy. Preferably, the subject is a patient suffering from cancer or an autoimmune disease. The subject can be a vertebrate, more preferably a mammal. Mammals include, but are not limited to, farm animals, sport animals, pets, primates, mice and rats. Preferably, a mammal is as a human, dog, cat, cow, pig, mouse, rat etc., particularly preferred, it is a human.

The present application has been mainly explained with reference to the use of blocking-reagents for reducing unspecific T cell activation. However, it is to be noted that the disclosure of the present invention applies in the same fashion to using a blocking-reagent in increasing the dosage of the administered bispecific antibody molecule compared to the dosage used without the blocking-reagent and/or CAR modified T cell or to using a blocking-reagent for reducing side-effects associated with T cell engaging therapies or to using a blocking-reagent in increasing the speed of the application of an administered bispecific antibody molecule and/or CAR modified T cell compared to the speed used without the blocking-reagent.

In accordance with the above disclosure, the present invention also provides a pharmaceutical composition that includes a blocking-reagent used in the present invention and optionally a pharmaceutically acceptable excipient.

In addition, the present invention also provides a pharmaceutical kit of parts that includes a blocking-reagent of the present invention and a bispecific antibody molecule as described herein in two separate parts, and optionally a pharmaceutically acceptable excipient. The present invention also provides a pharmaceutical kit of parts that includes a blocking-reagent of the present invention and a CAR modified T cell as described herein in two separate parts, and optionally a pharmaceutically acceptable excipient. In particular, the present invention provides for a pharmaceutical kit of parts, comprising in two separate parts:
a) a blocking-reagent, and
b) a bispecific antibody molecule,
   wherein the bispecific antibody molecule binds to
   i) a first antigen, and
   ii) a T cell receptor (TCR)/CD3 complex.

The first antigen may be any antigen, which is targeted by bispecific antibody therapy. Preferably said first antigen is an antigen associated with a target cell as described herein. A blocking-reagent present in the pharmaceutical kit of parts can be any blocking reagent as described herein.

Furthermore, the present invention relates to a pharmaceutical kit of parts, comprising, in two separate parts:
a) a blocking-reagent, and
b) a chimeric antigen receptor (CAR) modified T cell
   wherein the CAR comprises
i) an antibody molecule and
ii) a TCR/CD3 signaling domain.

In some embodiments, the antibody molecule comprises a binding site, which binds to an antigen associated with a target cell as described herein. A blocking-reagent present in such a pharmaceutical kit of parts can again be any blocking reagent as described herein.

In one embodiment of the present invention, the pharmaceutical composition or the pharmaceutical kit of parts is/are suitable for any route of administration as described above. Preferably, a blocking-reagent used in the present invention is applied in the same formulation as a bispecific antibody molecule or CAR modified T cell. However, the formulations for the blocking-reagent and the bispecific antibody molecule or CAR modified T cell can also be different. The pharmaceutical composition or the pharmaceutical kit of parts may be an aqueous solution, an oil-in water emulsion or a water-in-oil emulsion. The pharmaceutical composition or pharmaceutical kit of parts can contain a variety of conventional non-toxic pharmaceutically acceptable excipients or carriers, additives, and vehicles.

Accordingly, the blocking-reagent of the present invention as well as the bispecific antibody molecule or CAR modified T cell can be formulated into compositions using pharmaceutically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA). To prepare the pharmaceutical compositions, pharmaceutically inert inorganic or organic excipients can be used. To prepare e.g. pills, powders, gelatin capsules or suppositories, for example, lactose, talc, stearic acid and its salts, fats, waxes, solid or liquid polyols, natural and hardened oils can be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

The pharmaceutical composition or the pharmaceutical kit of parts may also contain additives, such as, for example, fillers, binders, wetting agents, glidants, stabilizers, preservatives, emulsifiers, and furthermore solvents or solubilizers or agents for achieving a depot effect. The latter is that fusion proteins may be incorporated into slow or sustained release or targeted delivery systems, such as liposomes and microcapsules.

The formulations can be sterilized by numerous means, including filtration through a bacteria-retaining filter, or by incorporating sterilizing agents in the form of sterile solid compositions which can be dissolved or dispersed in sterile water or other sterile medium just prior to use. Numerous possible applications for the blocking-reagent used in the present invention exist in medicine.

The present invention further relates to an *in vitro* method for evaluating unspecific T cell activation the method comprising
(i) contacting bystander cells and effector cells with bispecific antibody molecules as defined herein that do not bind to the bystander cells, or
(ii) contacting bystander cells and effector cells with CAR T cells as defined herein that do not bind to the bystander cells, and
(iii) measuring unspecific T cell activation.

The bystander cells, effector cells, bispecific antibodies or CARTs mentioned above can all be used in such a method as described herein. In particular, cells and set-up such as described in the Examples can be used to perform the *in vitro* method of the present invention. Illustrative examples of suitable bystander cells are HUVEC cells and SKW cells, in particular SKW 6.4 cells.

The unspecific T cell activation can be measured by the uptake of 3H-thymidine or determination of T cell activation markers such as CD69 expression by flow cytometry.

In some embodiments, in the *in vitro* method,
a) the bispecific antibody molecule comprises two binding sites
   i) wherein the first binding site binds to an antigen associated with a target cell, and
   ii) wherein the second binding site binds to a T cell receptor (TCR)/CD3 complex, and/or
b) the CAR comprises
   i) an antibody molecule comprising a binding site that binds to an antigen associated with a target cell, and
   ii) a TCR/CD3 signaling domain.

The present invention also relates to a use of a blocking-reagent used in the present invention or the pharmaceutical composition of the present invention or the pharmaceutical kit of parts of the present invention in the manufacture of a medicament for treating a subject having a disease. This disease can for example be a disease, which can be treated with a T cell engaging therapy. Preferably, the subject suffers from cancer or from an autoimmune disease.

The present invention also relates to a method of treating a disease in a subject, comprising the step of administering a blocking-reagent used in the present invention or a pharmaceutical composition of the present invention or a pharmaceutical kit of parts of the present invention to a subject in need thereof.

In addition the present invention relates to a method for reducing unspecific T cell activation in therapy, the therapy comprising administering to a subject a blocking-reagent that binds to a cell adhesion molecule or a cytokine and further administering to the subject a bispecific antibody molecule and/or a chimeric antigen receptor (CAR) modified T cell,
wherein the bispecific antibody molecule comprises two binding sites
i) wherein the first binding site binds to an antigen associated with a target cell, and
ii) wherein the second binding site binds to a T cell receptor (TCR)/CD3 complex and/or
   wherein the CAR comprises
iii) an antibody molecule comprising a binding site that binds to an antigen associated with a target cell, and
iv) a TCR/CD3 signaling domain.

Also the present invention relates to a method for reducing a side-effect in therapy, the therapy comprising
administering to a subject a blocking-reagent that binds to a cell adhesion molecule or a cytokine and further administering to the subject a bispecific antibody molecule and/or a chimeric antigen receptor (CAR) modified T cell,
wherein the bispecific antibody molecule comprises two binding sites
i) wherein the first binding site binds to an antigen associated with a target cell, and
ii) wherein the second binding site binds to a T cell receptor (TCR)/CD3 complex and/or
   wherein the CAR comprises
iii) an antibody molecule comprising a binding site that binds to an antigen associated with a target cell, and
iv) a TCR/CD3 signaling domain.

In some embodiments, the side-effect includes at least one of cytokine release syndrome, skin rash, hearing loss, uveitis, inflammatory colitis, tumor lysis syndrome, fever, chills, dyspnea, fatigue, tachycardia, hypertension, back pain, vomiting, seizures, encephalopathy, edema, aseptic meningitis, nausea or headache.

In addition, the present invention relates to a method for increasing the dosage of a bispecific antibody molecule and/or a chimeric antigen receptor (CAR) modified T cell in therapy, the therapy comprising
administering to a subject a blocking-reagent that binds to a cell adhesion molecule or a cytokine and further administering to the subject a bispecific antibody molecule and/or a chimeric antigen receptor (CAR) modified T cell,
wherein the bispecific antibody molecule comprises two binding sites
i) wherein the first binding site binds to an antigen associated with a target cell, and
ii) wherein the second binding site binds to a T cell receptor (TCR)/CD3 complex and/or
   wherein the CAR comprises
iii) an antibody molecule comprising a binding site that binds to an antigen associated with a target cell, and
iv) a TCR/CD3 signaling domain.

The present invention is further characterized by the following items:
1. Blocking-reagent for use in reducing unspecific T cell activation in therapy, the therapy comprising
   administering to a subject a bispecific antibody molecule and/or a chimeric antigen receptor (CAR) modified T cell,
   wherein the bispecific antibody molecule comprises two binding sites
   i) wherein the first binding site binds to an antigen associated with a target cell and
   ii) wherein the second binding site binds to a T cell receptor (TCR)/CD3 complex, on an effector cell and/or
      wherein the CAR comprises
   iii) an antibody molecule comprising a binding site that binds to an antigen associated with a target cell, and
   iv) a TCR/CD3 signaling domain.
2. Blocking-reagent for use of item 1, wherein the target cell expresses a tumor associated antigen (TAA) and/or an antigen associated with autoimmune diseases.
3. Blocking-reagent for use of item 2, wherein the TAA is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1R, (CD115), CD123, CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate blocking protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, Tie2, mesothelin, epithelial glycoprotein 2 (EGP2), epithelial glycoprotein 40 (EGP40), cancer antigen 72-4 (CA72-4), interleukin 13 receptor alpha-2 subunit, IL13Rα2, Ig kappa light chain (κ), GD3-ganglioside (GD3), GD2-ganglioside (GD2), CD171, NCAM, alpha folate receptor (αFR), Lewis(Y), fetal acetylcholine receptor (FAR), avian erythroblastic leukemia viral oncogene homolog 3 (ERBB3), avian erythroblastic leukemia viral oncogene homolog 4 (ERBB4), avian erythroblastic leukemia viral oncogene homolog 2 (ERBB2), hepatocyte growth factor receptor (HGFR/c-Met), claudin 18.2, claudin 3, claudin 4, claudin 1, claudin 12, claudin 2, claudin 5, claudin 8, claudin 7 and CD138.
4. Blocking-reagent for use of item 2, wherein the target cell expresses an antigen associated with autoimmune diseases, which antigen is selected from the group consisting of α4 subunit of α4β1 and α4β7 integrin, α4β7 integrin, BAFF, CD2, CD3, CD19, CD20, CD22, CD52, CD80, CD86.
5. Blocking-reagent for use of any of items 1-4, wherein the target cell is a tumor/cancer cell.
6. Blocking-reagent for use of any of items 1-5, wherein the effector cell is a T cell that carries the αβ- or the γδ-receptor, a cytotoxic T cell or a T helper cell.
7. Blocking-reagent for use of item 1 or 6, wherein the effector cell expresses TCR (alpha/beta) or TCR (gamma/delta).
8. Blocking- reagent for use of any of items 1-7, wherein the blocking-reagent reduces the activation of effector cells caused by a bystander cell.
9. Blocking-reagent for use of item 8, wherein the bystander cell is an endothelial cell or a lymphatic cell or any cell, capable of supporting T cell activation together with a soluble, monomeric molecule binding to the TCR/CD3 complex.
10. Blocking-reagent for use of any of items 1-9, wherein the blocking-reagent is selected from the group consisting of an antibody, a divalent antibody fragment, a monovalent antibody fragment, a proteinaceous binding molecule with antibody-like binding properties.
11. Blocking-reagent for use of item 10, wherein the divalent antibody fragment is an (Fab)₂-fragment, or a divalent single-chain Fv fragment.
12. Blocking-reagent for use of item 10, wherein the monovalent antibody fragment is selected from the group consisting of a Fab fragment, a Fv fragment, and a single-chain Fv fragment (scFv).
13. Blocking-reagent for use of item 10, wherein the proteinaceous binding molecule with antibody-like binding properties is selected from the group of an aptamer, a mutein based on a polypeptide of the lipocalin family, a glubody, a protein based on the ankyrin scaffold, a protein based on the crystalline scaffold, an adnectin, and an avimer.
14. Blocking-reagent for use of any of items 1-13, wherein the blocking-reagent binds to a cell adhesion molecule or a cytokine.
15. Blocking-reagent for use of item 14, wherein the cell adhesion molecule is selected the group consisting of CD18, CD11 a, CD11 b, CD11 c, ICAM-1 (CD54), ICAM-2 (CD102), LFA1, LFA2 (CD2), CD58, CD86, CD80, OX-40 (CD134), 4-1 BB and/or LICOS (CD275) and/or the cytokine is selected from TNFalpha.
16. Blocking-reagent for use of item 15, wherein the blocking-reagent is selected from the group consisting of an anti-CD18 antibody, an anti-CD11b antibody, an anti-CD11c antibody, an anti-LFA1 antibody, an anti-CD275 antibody, an anti-CD54 antibody, an anti-CD102 antibody, an anti-CD86 antibody, an anti-CD2 antibody, and an anti-TNFalpha antibody, an anti-CD11a antibody.
17. Blocking-reagent for use of item 16, wherein the blocking-reagent is selected from the group consisting of an anti-CD18 antibody, an anti-CD275 antibody, an anti-CD54 antibody, an anti-TNFalpha antibody, a combination of an anti-CD54 and an anti-CD102 antibody and/or an anti-CD2 antibody, preferably the antibody is an anti-CD18 antibody.
18. Blocking-reagent for use of any of items 1-17, wherein the first binding site of the bispecific antibody molecule binds to a tumor associated antigen (TAA) as defined in item 3.
19. Blocking-reagent for use of any of items 1-18, wherein the second binding site of the bispecific antibody molecule binds to CD3.
20. Blocking-reagent for use of item 19, wherein the bispecific antibody molecule comprises a binding site of the UCHT1 antibody, which has a sequence identity of at least 80%, or at least 85 %, or at least 90 %, or at least 95 %, or at least 98 %, or at least 99 % or 100 % to SEQ ID NO. 1 (the sequence of the light chain of the variable domain of UCHT-1).
21. Blocking-reagent for use of item 19 or 20, wherein the bispecific antibody molecule comprises a binding site of the UCHT-1 antibody, which has a sequence identity of at least 80%, or at least 85 %, or at least 90 %, or at least 95 %, or at least 98 %, or at least 99 % or 100 % to SEQ ID NO. 2 (the sequence of the heavy chain of the variable domain of UCHT-1).
22. Blocking-reagent for use of item 19, wherein the bispecific antibody molecule comprises a binding site of the OKT3 antibody, which has a sequence identity of at least 80%, or at least 85 %, or at least 90 %, or at least 95 %, or at least 98 %, or at least 99 % or 100 % to SEQ ID NO. 3 (the sequence of the light chain of the variable domain of OKT3).
23. Blocking-reagent for use of any of items 19 or 22, wherein the bispecific antibody molecule comprises a binding site of the OKT3 antibody, which has a sequence identity of at least 80%, or at least 85 %, or at least 90 %, or at least 95 %, or at least 98 %, or at least 99 % or 100 % to SEQ ID NO. 4 (the sequence of the heavy chain of the variable domain of OKT3).
24. Blocking-reagent for use of any of items 1-23, wherein the CAR comprises an antibody molecule, which is single-specific or bispecific.
25. Blocking-reagent for use of item 24, wherein the CAR comprises an antibody molecule comprising a binding site that binds to a TAA as defined in item 3.
26. Blocking-reagent for use of item 24, wherein the TAA is selected from the group consisting of CD19, CD20, CD30, CD33, CD138, Lewis Y, EGFR and Ig kappa light chain (κ).
27. Blocking-reagent for use of any of items 24-26, wherein the CAR comprises an antibody molecule, which antibody molecule preferably comprises a scFv.
28. Blocking-reagent for use of item 27, wherein the CAR comprises a scFv, which is derived from a TAA-specific monoclonal antibody.
29. Blocking-reagent for use of any of items 1-28, wherein the TCR/CD3 signaling domain comprises a CD3ζ domain.
30. Blocking-reagent for use of item 29, wherein the TCR/CD3 signaling domain comprises a CD3ζ domain and one co-stimulatory domain.
31. Blocking-reagent for use of item 30, wherein the co-stimulatory domain is selected from the group consisting of 4-1 BB or CD28.
32. Blocking-reagent for use of item 29, wherein the TCR/CD3 signaling domain comprises a CD3ζ domain and two co-stimulatory domains.
33. Blocking-reagent for use of item 32, wherein the co-stimulatory domains are selected from the group consisting of 4-1 BB, CD28, CD27, OX40 or ICOS.
34. Blocking-reagent for use of any of items 29-33, wherein the TCR/CD3 signaling domain is selected from the group consisting of 4-1BB-CD3ζ, CD28-CD3ζ, CD28-4-1BB-CD3ζ, CD3ζ, CD137- CD3ζ, anti-Lewis Y-CD28-CD3ζ.
35. Blocking-reagent for use of any of items 1-34, wherein the blocking-reagent and the bispecific antibody molecule and/or CAR modified T cell are administered simultaneously or sequentially.
36. Blocking-reagent for use of item 35, wherein the blocking-reagent is administered before the bispecific antibody molecule is administered.
37. Blocking-reagent for use of 35, wherein the blocking-reagent is administered after the bispecific antibody molecule has been administered.
38. Blocking-reagent for use of any of items 1-37, wherein the blocking-reagent and the CAR modified T cell are administered simultaneously or sequentially.
39. Blocking-reagent for use of item 38, wherein the blocking-reagent is administered before the CAR modified T cell is administered.
40. Blocking-reagent for use of item 38, wherein the blocking-reagent is administered after the CAR modified T cell has been administered.
41. Blocking-reagent for use of any of items 1-40, wherein therapy is a therapy for treating a proliferatory disease or an autoimmune disease.
42. Blocking-reagent for use of item 41, wherein the proliferatory disease is selected from the group consisting of hemopoetic malignancies, such as acute and chronic myeloic and lymphatic leukemias, as well as lymphomas, solid tumors such as tumors of the gastrointestinal tract, lung, kidney, prostate, breast, brain, ovary, uterus, mesenchymal tumors and melanoma.
43. Blocking-reagent for use of item 41, wherein the autoimmune disease is selected from the group consisting of Systemic lupus erythematosus (SLE), Goodpasture's syndrome, Sarcoidosis, Scleroderma, Rheumatoid arthritis, Dermatomyositis, Sjögren's Syndrome, Scleroderma, Dermatomyositis, Psoriasis, Vitiligo, Alopecia areata, Type 1 diabetes mellitus, Autoimmune pancreatitis, Hashimoto's thyroiditis, Addison's disease, Multiple sclerosis, Myasthenia gravis, Polyarteritis nodosa, Idiopathic thrombocytopenic purpura, Hemolytic anemia, Antiphospholipid antibody syndrome, Pernicious anemia, Gastrointestinal diseases, Celiac disease, Inflammatory bowel disease, Autoimmune hepatitis or Primary biliary cirrhosis.
44. Blocking-reagent for use of any of items 1-43, wherein side-effects of the therapy are reduced.
45. Blocking-reagent for use of item 44, wherein the side-effects include at least one of cytokine release syndrome, skin rash, hearing loss, uveitis, inflammatory colitis, tumor lysis syndrome, fever, chills, dyspnea, fatigue, tachycardia, hypertension, back pain, vomiting, seizures, encephalopathy, edema, aseptic meningitis, nausea or headache.
46. Blocking-reagent for use of any of items 1-45, wherein in therapy the dosage of the administered bispecific antibody and/or CAR modified T cell is increased compared to the dosage used without the blocking-reagent.
47. Blocking-reagent for use of any of items 1-46, wherein the subject is a vertebrate, preferably a human being.
48. A pharmaceutical kit of parts, comprising in two separate parts:
   a) a blocking-reagent, and
   b) a bispecific antibody molecule,
   wherein the bispecific antibody molecule binds to
   i) a first antigen, and
   ii) a T cell receptor (TCR)/CD3 complex.
49. A pharmaceutical kit of parts, comprising, in two separate parts:
   a) a blocking-reagent, and
   b) a chimeric antigen receptor (CAR) modified T cell
   wherein the CAR comprises
   i) an antibody molecule and
   ii) a TCR/CD3 signaling domain.
50. An *in vitro* method for evaluating unspecific T cell activation, the method comprising
   (i) contacting bystander cells and effector cells with bispecific antibody molecules as defined in item 1 that do not bind to the bystander cells, or
   (ii) contacting bystander cells and effector cells with CAR T cells as defined in item 1 that do not bind to the bystander cells, and
   (iii) measuring unspecific T cell activation.
51. In vitro method of item 50, wherein the unspecific T cell activation is measured by the uptake of 3H-thymidine or determination of T cell activation markers such as CD69 expression by flow cytometry.
52. In vitro method of item 51, wherein
   a) the bispecific antibody molecule comprises two binding sites
      i) wherein the first binding site binds to an antigen associated with a target cell, and
      ii) wherein the second binding site binds to a T cell receptor (TCR)/CD3 complex, and/or
   b) the CAR comprises i) an antibody molecule comprising a binding site that binds to an antigen associated with a target cell, and
   ii) a TCR/CD3 signaling domain.
53. Use of the blocking-reagent as defined in any of items 10-17 or the pharmaceutical composition kit of parts of any of items 48 or 49 in the manufacture of a medicament for treating a subject having a disease.
54. A method of treating a disease in a subject, comprising the step of administering the blocking-reagent as defined in any of items 1 or 10-17 or the pharmaceutical composition kit of parts of any of items 48 or 49 to a subject in need thereof.
55. A method for reducing unspecific T cell activation in therapy, the therapy comprising
   administering to a subject a blocking-reagent that binds to a cell adhesion molecule or a cytokine and further administering to the subject a bispecific antibody molecule and/or a chimeric antigen receptor (CAR) modified T cell, wherein the bispecific antibody molecule comprises two binding sites
   i) wherein the first binding site binds to an antigen associated with a target cell, and
   ii) wherein the second binding site binds to a T cell receptor (TCR)/CD3 complex and/or
      wherein the CAR comprises
   iii) an antibody molecule comprising a binding site that binds to an antigen associated with a target cell, and
   iv) a TCR/CD3 signaling domain.
56. A method for reducing a side-effect in therapy, the therapy comprising
   administering to a subject a blocking-reagent that binds to a cell adhesion molecule or a cytokine and further administering to the subject a bispecific antibody molecule and/or a chimeric antigen receptor (CAR) modified T cell, wherein the bispecific antibody molecule comprises two binding sites
   i) wherein the first binding site binds to an antigen associated with a target cell, and
   ii) wherein the second binding site binds to a T cell receptor (TCR)/CD3 complex and/or
      wherein the CAR comprises
   iii) an antibody molecule comprising a binding site that binds to an antigen associated with a target cell, and
   iv) a TCR/CD3 signaling domain.
57. The method of item 56, wherein the side-effect includes at least one of cytokine release syndrome, skin rash, hearing loss, uveitis, inflammatory colitis, tumor lysis syndrome, fever, chills, dyspnea, fatigue, tachycardia, hypertension, back pain, vomiting, seizures, encephalopathy, edema, aseptic meningitis, nausea or headache.
58. A method for increasing the dosage of a bispecific antibody molecule and/or a chimeric antigen receptor (CAR) modified T cell in therapy, the therapy comprising
   administering to a subject a blocking-reagent that binds to a cell adhesion molecule or a cytokine and further administering to the subject a bispecific antibody molecule and/or a chimeric antigen receptor (CAR) modified T cell, wherein the bispecific antibody molecule comprises two binding sites
   i) wherein the first binding site binds to an antigen associated with a target cell, and
   ii) wherein the second binding site binds to a T cell receptor (TCR)/CD3 complex and/or
      wherein the CAR comprises
   iii) an antibody molecule comprising a binding site that binds to an antigen associated with a target cell, and
   iv) a TCR/CD3 signaling domain.

The invention is further illustrated by the following non-limiting Examples.

### EXAMPLE I

It is widely held that T cell activation requires a multivalent CD3 stimulus formed after binding of a bispecific antibody molecules with specificities to a target cell associated antigen (e.g. TAA) and the TCR/CD3 complex. However, this bispecific antibody molecule does not always bind to its two targets. It can be that it only binds either to the target cell associated antigen (e.g. TAA) or the TCR/CD3 complex (monovalent stimulus). However, such a monovalent stimulus, as provided by most current bispecific antibody molecules in solution, should not activate T cells. This is the basis of the concept of target cell restricted T cell activation with bispecific antibody molecules as outlined above (Jung G et al., (1986) and (1988) cited above; Brischwein K, Parr L, Pflanz S, Volkland J, Lumsden J, Klinger M, Locher M, Hammond SA, Kiener P, Kufer P, Schlereth B, Baeuerle PA. Strictly target cell-dependent activation of T cells by bispecific single-chain antibody constructs of the BiTE class. J Immunother. 2007; 30:798-807).

However more recent data suggested that a monovalent TCR/CD3 stimulus in the absence of target cells may lead to some unspecific T cell activation. This type of target cell independent T cell activation (true "off target" activation) by the CD3 part of a bispecific antibody molecule in the absence of any target cell to which the antibody binds could be exaggerated in the presence of stimulatory bystander cells (SBCs), e.g lymphatic or endothelial cells, expressing certain costimulatory-oder adhesion molecules.

To test this hypothesis experiments were performed to test for an off target T cell activation mediated by SBCs in the presence of bispecific antibody molecules. To this end, PBMCs were isolated from heparinized blood of normal donors and seeded in 96 well plates (100.000 per well) together with different irradiated bystander cells, such as SKW6.4 lymphoblastoid cells, JY lymphoblastoid cells, umbilical vein endothelial cells (HUVECs) or lymphoblastoid NALM16 cells (100.000 per well). These "PMBC-SBC co-cultures" were compared to a control PMBC cultures without antibody (indicated as "PBMC" on the y axis) or to cultures containing only bystander cells (indicated as "-" on the y axis).

To analyze the unspecific T cell activation of a bispecific antibody molecule on these co-cultures, a bispecific "Fabsc"-antibody molecule as described in International patent application 2013/092001 with PSMA X CD3-specificity (1 µg/ml) was added to these different cell cultures (indicated as "PBMC+NP-CU" on the y-axis). The single chain Fv fragment of this PSMA x CD3 bispecific "Fabsc"-antibody molecule binds to CD3, while the Fab fragment of this antibody molecule binds to PSMA. Notably, the prostate specific membrane antigen (PSMA) to which the bispecific antibody molecule binds is neither expressed on PBMCs nor on the bystander cells. Therefore, the bispecific PSMA X CD3 antibody molecule can only bind to effector cells in the PBMC culture via its CD3-targeting effector part. Therefore, the PSMA x CD3 antibody molecule will not bind any of the cells present in the PMBC-SBC co-culture. In other words, the co-culture lacks any target cells.

To understand the effect of SBCs on off target cell T cell activation in the presence of the bispecific antibody molecules better, as an additional control, an intact, anti-CD3 antibody molecule was added to different wells containing the co-cultures (indicated as "PBMC+UCHT1" on the y-axis). This antibody serves as a positive control, since it is well established that CD3 antibodies by binding to monocyte Fc-receptors induce maximal polyclonal T cell activation in PBMC cultures. After 2 days, cells were pulsed with 3H-thymidine (0.5 µCi per well), harvested 20 hours later on filter mats and counted in a scintillation counter. The thymidine uptake is commonly used to measure cell proliferation as counts per minute (CPM). Therefore, the measured cell proliferation is also an indication of immune cell activation. As the experimental set-up is such that only T cells are activated via the CD3-targeting effector part of the bispecific antibody molecule, in this experiment specifically T cell activation is measured via cpm analysis. The dark grey bars indicate the effect of the bispecific antibody molecules on unspecific T cell activation. The bispecific antibody molecule increased proliferation in cultures containing PBMC and SKW6.4- and JY lymphoblastoid cells as well as human umbilical vein endothelial cells (HUVECs). However, proliferation was not increased in the control culture or in the co-culture of lymphoblastoid NALM16 cells and PBMC.

On the other hand, the anti-CD3 antibody molecule lead to an increase in cell proliferation in all the different cultures analyzed (Fig. 1B). On the contrary, cultures comprising only one cell type (PBMC or SBC cells) did not resume a high amount of cell proliferation in the presence of bispecific antibody molecules. Thus, bystander cells such as the lymphoblastoid cell linesSKW6.4 or JY, as well as human umbilical vein endothelial cells (HUVECs) but not others, such as NALM 16 cells, can serve as SBCs for bispecific antibody molecules (Fig. 1 B). The pattern depicted in Figure 1B has been observed reproducibly with PBMCs from different donors.

These bystander cells could be endothelial cells and lymphatic cells in the lymph node compartment under physiological conditions. That the interaction with endothelial cells contributes to T cell activation by bispecific TAAXCD3 Fab₂ antibody molecules has also been demonstrated by Molema et al., (2000) (cited above). Transendothelial migration of T cells during in vivo application of bispecific antibody molecules is also suggested by the rapid - albeit transient - lymphocyte depletion observed during treatment (Klinger et al., (2012) cited above). This phenomenon most likely contributes significantly to unspecific T cell activation induced by these bispecific antibody molecules.

Notably, what has been said above does not only apply to T cells coated with a bispecific target cell associated antigen (e.g. TAA) X TCR/CD3 complex-antibody molecules but also to T cells transfected with a chimeric antigen receptor (CAR). CARs are hybrid molecules comprising an antibody single chain molecule comprising a binding site that binds to an antigen associated with a target cell as an extracellular recognition unit and an intracellular signaling domain derived from the T cell receptor (TCR) associated CD3 molecule (Fig. 2). Thus, as hinted above, T cells carrying a CAR that contains for example an anti-TAA antibody molecule as a recognition unit, closely resembles cells that are coated with e.g. a bispecific TAA X CD3 antibody molecule (Figure 2).

As for the effect of SBCs on unspecific T cell activation, SBCs will presumably also contribute to unspecific T cell activation of CAR T cells. This is also indicated observations of life threatening cytokine release syndromes in patients receiving large numbers of CAR T cells specific for Her2, an antigen rather specifically expressed on Her2-positive mammary carcinoma cells (Morgan et al., (2010) cited herein).

Thus, unspecific T cell activation is considerable upon usage of bispecific antibody molecules or CAR modified T cells. Stimulating bystander cells (SBC) dramatically enhances unspecific T cell activation of bispecific TAA X CD3 antibody molecules in the absence of target cells ("off target activation").

### EXAMPLE II

In the experiments depicted in Figure 3, the set up was as that described for Fig. 1B and in Example 1. However, now blocking-reagents to various adhesion molecules and cytokines were added to the PBMC-SBC co-cultures. With this experimental seting the influence of blocking-reagents on off target cell activation is measured. Furthermore, due to the design of the experiment, a decrease in proliferation (compared to the isotype control) indicates that unspecific T cell activation is reduced.

Fig. 3 (A) shows PBMC-SBC co-cultures containing PBMCs and human umbilical vein endothelial cells (HUVECs) purchased by Promocell, (Heidelberg, Germany). The addition of the control antibody F19, directed to the fibroblast actvating protein (FAP) provides the extent of base-line cell proliferation (about 30000 cpm in Figures 1A, C, D) that is comparable to proliferation in the absence of antibodies. On the contrary, addition of anti-TNFalpha antibody molecules (aTNFa/infliximab) resulted in a slight decrease in proliferation (to about 20000 cpm). Notably, the addition of a combination of an anti-CD54 with anti-CD102 antibody molecules (aCD54+aCD102/ICAM1/2), anti-CD18 antibody molecules (aCD-18/integrin b2) or anti-CD2 antibodies (aCD2/LFA-2) resulted in a marked decrease in cell proliferation (to about 10000 cpm or less; Figure 1A). Similar results were obtained with PBMCs from four different healthy donors.

Fig. 3 (B) depicts PBMC-SBC cultures containing PBMCs and human umbilical vein endothelial cells (HUVECs). In these co-cultures, addition of the control FAP antibody F19 provides the amount of base-line cell proliferation (about 10000 cpm). The addition of an anti-IL-6R antibody molecule (alL-6/tocilizumab) or an anti-CD11a antibody molecule (aCD11a/LFA-1) showed an increase in cell proliferation (about 15000 cpm), while the addition of an anti-CD275 (aCD275/LICOS) or an anti-CD54 antibody molecule (ICAM-1) resulted in a slight decrease in proliferation (about 6500 cpm). Notably, the addition of an anti-CD18 antibody molecule (aCD18/integrin b2) resulted in a complete block of proliferation. Again, similar results were obtained with PBMCs from four different healthy donors.

Fig. 3 (C) shows PBMC-SBC co-cultures containing PBMCs and SKW cells. The addition of the control FAP (F19, control, ATCC) provided the amount of base-line cell proliferation (about 30000 cpm). The addition of an anti-CD275 antibody molecule (aCD275/LICOS) or an anti-CD86 antibody molecule (aCD86) showed a slight decrease in cell proliferation (about 25000 cpm), while the addition of an anti-CD54 antibody molecule (aCD54/ICAM-1) resulted in a marked decrease in proliferation (about 20000 cpm). Notably, the addition of an anti-CD18 antibody molecule (aCD18/integrin b2) resulted in an almost complete block of proliferation. Similar results were obtained with PBMCs from four different healthy donors.

Fig. 3 (D) depicts again co-cultures of PBMC and SKW cells. Here, the isotype control (F19) showed a proliferation of about 25000-30000 cpm, while the addition of an anti-CD2 antibody molecule resulted in a slight decrease in proliferation (aCD2/LFA-2). Again, the addition of an anti-CD18 antibody molecule (aCD18/integrin b2/TS 1/18 antibody) showed an almost complete block of proliferation. Similar results were obtained with PBMCs from four different healthy donors.

In summary, Figure 3 shows that off target T cell activation by bispecific antibody molecules and SBCs (HUVECs or SKW6.4) was not affected by the control antibody molecule and an IL-6 antibody molecule, moderately inhibited by antibody molecules directed to CD11a, LICOS, and TNF, markedly inhibited by a combination of ICAM-1/2 antibody molecules and a CD2 antibody molecule. An antibody molecule directed to CD18 completely blocked the SBC effect at concentrations ≥1 µg/ml.

These results proved that unspecific T cell activation by bispecific TAA X CD3 antibody molecules and SBCs is inhibited by antibody molecules to certain adhesion molecules and cytokines (blocking of "off target activation"). Without being bound to theory it is believed that reduction of unspecific T cell activation is achieved by reducing the association between SBCs and T cells, in general and in particular by reducing the interaction of activated T cells and endothelial cells.

### EXAMPLE III

Also in Example 3 (Fig. 4) the experimental set up was identical to that described in Fig. 1B and Example 1 except that the bispecific Fabsc antibody molecule added recognized a target antigen expressed on the SBCs. The target antigens expressed on the bystander cells were Endoglin (CD105) on HUVEC cells (A), PSMA on RV1 cells (B), CD19 on SKW cells (C) and FLT3 on NALM 16 cells (D). Thus, different bispecific antibody molecules were added to the different co-cultures, which bind to the respective bystander cells present in these cultures, namely CD105xCD3 (A), PSMA X CD3 (B), CD19 X CD3 (C) and FLT3XCD3 (D) antibody molecules. Furthermore, in each co-culture the effect of different blocking-reagents that were shown to reduce the off target (unspecific) T cell activation (in Example 2) were analyzed.

Thus, in contrast, to the experiments shown in Figure 1 and 3, now in Figure 4 the on-target activation of T cells is depicted. That means that in this case only blocking-reagents that did not block the "on target cell" proliferation (Fig. 4), but did block "off target" cell proliferation (Fig. 3) are the most interesting blocking-reagents for the purposes of the present invention.

As can be seen in Figure 3(A) the anti-CD54 antibody molecule (aCD54/ICAM-1), the anti-CD18 antibody molecule (aCD18/Integrin b2) and the anti-CD11a antibody molecule (aCD11a/LFA-1) performed equally to the isotype control (F19). Thus, these blocking-reagents did not have an effect on cell proliferation (about 1,2e+5 cpm). On the contrary, the addition of the anti-TNFa-antibody molecule (aTNFa/infliximab) moderately decreased the proliferation of T cells (and therefore also the specific T cell activation) in this experiment (from approx. 120.000 to 80.000 cpm).

In Figures 4 (B) and 4 (C) the blocking-reagents, namely the anti-CD54 antibody molecule (aCD18/Integrin b2) and the anti-CD18 antibody molecule (aCD54/ICAM-1), did not significantly bock on target T cell proliferation (about 60000 cpm in (B) and (C)).

Notably, in Fig. 4 (D) the anti-CD18 antibody molecule (anti-CD18), the anti-CD54 antibody molecule (anti-CD54), and the anti-TNFa antibody molecule (infliximab) all did not influence the proliferation notably different from the isotype control (F19) (all about 70000-80000 cpm). Only the anti-CD2 antibody molecule decreased the proliferation (about 40000 cpm).

In all experiments of Figure 4, similar results were obtained with PBMCs from three different healthy donors. T cell activation in the presence of a bispecific antibody molecule directed to antigens expressed on SBCs (Endoglin X CD3, PSMA X CD3, CD19 X CD3 and FLT3 X CD3 in the case of HUVECs, RV1-, SKW- and Nalm16-cells, respectively) was not blocked by CD18 antibody molecules (Fig. 4). This indicates that only off target T cell activation by SBCs was blocked, whereas activation in the presence of target cells and bispecific antibody molecules remained unaffected.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of certain embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims.

### REFERENCE LIST (SCIENTIFIC REFERENCES)

1) Staerz UD, Kanagawa O, Bevan MJ. Hybrid antibodies can target sites for attack by T cells. Nature 1985; 314:628-631.
2) Perez P, Hoffman RW, Shaw S, Bluestone JA, Segal DM. Specific targeting of cytotoxic T cells by anti-T3 linked to anti-target cell antibody. Nature 1985; 316:354-356.
3) Jung G, Honsik CJ, Reisfeld RA and Müller-Eberhard HJ. Activation of human peripheral blood mononuclear cells by anti-T3: Killing of tumor target cells coated with anti-target X anti-T3-conjugates. Proc Natl Acad Sci USA 1986; 83:4479-4483.
4) Jung G and Müller-Eberhard HJ. An in vitro model for tumor immunotherapy with antibody-heteroconjugates. Immunol Today 1988; 9:257-260.
5) Jung G, Freimann U, v.Marschall Z, Reisfeld RA and Wilmanns W. Target cell induced T cell activation with bi- and trispecific antibody molecules. Eur J Immunol 1991; 21:2431-2435.
6) Bargou R, Leo E, Zugmaier G et al. Tumor regression in cancer patients by very low doses of a T cell-engaging antibody. Science 2008; 321:974-977
7) Adams GP, Weiner LM. Monoclonal antibody therapy of cancer. Nat Biotechnol. 2005; 23:1147-57.
8) Topp MS, Kufer P, Gokbuget N et al. Targeted therapy with the T cell-engaging antibody blinatumomab of chemotherapy-refractory minimal residual disease in B-lineage acute lymphoblastic leukemia patients results in high response rate and prolonged leukemia-free survival. J Clin Oncol 2011; 29:2493-2498.
9) Kroesen BJ, Buter J, Sleijfer DT et al. Phase I study of intravenously applied bispecific antibody in renal cell cancer patients receiving subcutaneous interleukin 2. Br J Cancer 1994; 70:652-661.
10) Tibben JG, Boerman OC, Massuger LF et al. Pharmacokinetics, biodistribution and biological effects of intravenously administered bispecific monoclonal antibody OC/TR F(ab')2 in ovarian carcinoma patients. Int J Cancer 1996; 66:477-483.
11) Brischwein K, Parr L, Pflanz S, Volkland J, Lumsden J, Klinger M, Locher M, Hammond SA, Kiener P, Kufer P, Schlereth B, Baeuerle PA. Strictly target cell-dependent activation of T cells by bispecific single-chain antibody constructs of the BiTE class. J Immunother. 2007; 30:798-807.
12) Molema G, Tervaert JW, Kroesen BJ, Helfrich W, Meijer DK, de Leij LF. CD3 directed bispecific antibodies induce increased lymphocyte-endothelial cell interactions in vitro. Br J Cancer 2000; 82:472-479.
13) Klinger M, Brandl C, Zugmaier G, Hijazi Y, Bargou RC, Topp MS, Gökbuget N, Neumann S, Goebeler M, Viardot A, Stelljes M, Brüggemann M, Hoelzer D, Degenhard E, Nagorsen D, Baeuerle PA, Wolf A, Kufer P. Immunopharmacologic response of patients with B-lineage acute lymphoblastic leukemia to continuous infusion of T cell-engaging CD19/CD3-bispecific BiTE antibody blinatumomab. Blood 2012; 119:6226-33.
14) Brentjens RJ, Davila ML, Riviere I, Park J, Wang X, Cowell LG, Bartido S, Stefanski J, Taylor C, Olszewska M, Borquez-Ojeda O, Qu J, Wasielewska T, He Q, Bernal Y, Rijo IV, Hedvat C, Kobos R, Curran K, Steinherz P, Jurcic J, Rosenblat T, Maslak P, Frattini M, Sadelain M. CD19-targeted T cells rapidly induce molecular remissions in adults with chemotherapy-refractory acute lymphoblastic leukemia. Sci Transl Med 2013; 5:1-9.
15) Maus MV, Grupp SA, Porter DL, June CH. Antibody-modified T cells: CARs take the front seat for hematologic malignancies. Blood 2014; 123:2625-35.
16) Ulbrich H, Eriksson EE, Lindbom L. Leukocyte and endothelial cell adhesion molecules as targets for therapeutic interventions in inflammatory disease. Trends Pharmacol Sci 2003; 24:640-647.
17) Faxon DP, Gibbons RJ, Chronos NA, Gurbel PA, Sheehan F; HALT-MI Investigators. The effect of blockade of the CD11/CD18 integrin receptor on infarct size in patients with acute myocardial infarction treated with direct angioplasty: the results of the HALT-MI study. J Am Coll Cardiol 2002; 40:1199-1204.
18) Baran KW, Nguyen M, McKendall GR, Lambrew CT, Dykstra G, Palmeri ST, Gibbons RJ, Borzak S, Sobel BE, Gourlay SG, Rundle AC, Gibson CM, Barron HV; Limitation of Myocardial Infarction Following Thrombolysis in Acute Myocardial Infarction (LIMIT AMI) Study Group. Double-blind, randomized trial of an anti-CD18 antibody in conjunction with recombinant tissue plasminogen activator for acute myocardial infarction: limitation of myocardial infarction following thrombolysis in acute myocardial infarction (LIMIT AMI) study. Circulation 2001; 104:2778-2783.
19) Rusnak JM, Kopecky SL, Clements IP, Gibbons RJ, Holland AE, Peterman HS, Martin JS, Saoud JB, Feldman RL, Breisblatt WM, Simons M, Gessler CJ Jr, Yu AS. An anti-CD11/CD18 monoclonal antibody in patients with acute myocardial infarction having percutaneous transluminal coronary angioplasty (the FESTIVAL study). Am J Cardiol 2001; 88:482-487.
20) Rhee P, Morris J, Durham R, Hauser C, Cipolle M, Wilson R, Luchette F, McSwain N, Miller R. Recombinant humanized monoclonal antibody against CD18 (rhuMAb CD18) in traumatic hemorrhagic shock: results of a phase II clinical trial. Traumatic Shock Group. J Trauma 2000; 49:611-619.
21) Bowen JD, Petersdorf SH, Richards TL, Maravilla KR, Dale DC, Price TH, St John TP, Yu AS. Phase I study of a humanized anti-CD11/CD18 monoclonal antibody in multiple sclerosis. Clin Pharmacol Ther 1998; 64:339-346.
22) Morgan RA, Yang JC, Kitano M, Dudley ME, Laurencot CM, Rosenberg SA. Case report of a serious adverse event following the administration of T cells transduced with a chimeric antigen receptor recognizing ERBB2. Mol Ther 2010 Vol 18, No. 4, 843-851.
23) Barrett DM1, Teachey DT, Grupp SA. Toxicity management for patients receiving novel T cell engaging therapies. Curr Opin Pediatr. 2014 Feb;26(1):43-9.
24) Ramos CA, Dotti G. Chimeric antigen receptor (CAR)-engineered lymphocytes for cancer therapy. Expert Opin Biol Ther. 2011 Jul;11(7):855-73
25) III CR, Gonzales JN, Houtz EK, Ludwig JR, Melcher ED, Hale JE, Pourmand R, Keivens VM, Myers L, Beidler K, Stuart P, Cheng S, Radhakrishnan R. Design and construction of a hybrid immunoglobulin domain with properties of both heavy and light chain variable regions. Protein Eng. 1997 Aug;10(8):949-57.
26) Martin F, Toniatti C, Salvati AL, Venturini S, Ciliberto G, Cortese R, Sollazzo M. The affinity-selection of a minibody polypeptide inhibitor of human interleukin-6. EMBO J. 1994 Nov 15;13(22):5303-9.
27) Traunecker A, Lanzavecchia A, Karjalainen K. Bispecific single chain molecules (Janusins) target cytotoxic lymphocytes on HIV infected cells. EMBO J. 1991 Dec;10(12):3655-9.
28) Traunecker A, Lanzavecchia A, Karjalainen K. Janusin: new molecular design for bispecific reagents. Int J Cancer Suppl. 1992;7:51-2.
29) Silverman J, Liu Q, Bakker A, To W, Duguay A, Alba BM, Smith R, Rivas A, Li P, Le H, Whitehorn E, Moore KW, Swimmer C, Perlroth V, Vogt M, Kolkman J, Stemmer WP. Multivalent avimer proteins evolved by exon shuffling of a family of human receptor domains. Nat Biotechnol. 2005 Dec;23(12):1556-61. Epub 2005 Nov 20.
30) Sela M. Antigenicity: some molecular aspects. Science. 1969 Dec 12;166(3911):1365-74.
31) Holt LJ, Herring C, Jespers LS, Woolven BP, Tomlinson IM. Domain antibodies: proteins for therapy. Trends Biotechnol. 2003 Nov;21(11):484-90.
32) Roeland Lamerisa, Renée C.G. de Bruina, Famke L. Schneidersa, Paul M.P. van Bergen en Henegouwenb, Henk M.W. Verheula, Tanja D. de Gruijla, Hans J. van der Vliet Bispecific antibody platforms for cancer immunotherapy. Crit Rev Oncol Hematol. 2014 Aug 20. pii: S1040-8428(14)00135-8. doi: 10.1016/j.critrevonc.2014.08.003. [Epub ahead of print]
33) Morrison SL, Johnson MJ, Herzenberg LA, Oi VT. Chimeric human antibody molecules: mouse antigen-binding domains with human constant region domains. Proc Natl Acad Sci U S A. 1984 Nov;81(21):6851-5.
34) Jones PT, Dear PH, Foote J, Neuberger MS, Winter G. Replacing the complementarity-determining regions in a human antibody with those from a mouse. Nature. 1986 May 29-Jun 4;321(6069):522-5.
35) Verhoeyen M, Milstein C, Winter G. Reshaping human antibodies: grafting an antilysozyme activity. Science. 1988 Mar 25;239(4847):1534-6.
36) Co MS1, Queen C. Humanized antibodies for therapy. Nature. 1991 Jun 6;351(6326):501-2.
37) Padlan EA. A possible procedure for reducing the immunogenicity of antibody variable domains while preserving their ligand-binding properties. Mol Immunol. 1991 Apr-May;28(4-5):489-98.
38) Pedersen JT, Henry AH, Searle SJ, Guild BC, Roguska M, Rees AR. Comparison of surface accessible residues in human and murine immunoglobulin Fv domains. Implication for humanization of murine antibodies. J Mol Biol. 1994 Jan 21 ;235(3):959-73.
39) Mark G. E. et al (1994) in Handbook of Experimental Pharmacology vol. 113: The pharmacology of monoclonal Antibodies, Springer-Verlag, pp 105-134.
40) Skerra, A. Use of the tetracycline promoter for the tightly regulated production of a murine antibody molecule in Escherichia coli, Gene (1994) 151, 131-135.
41) Skerra, A. A general vector, pASK84, for cloning, bacterial production, and single-step purification of antibody Fab fragments, Gene (1994)141, 79-8.
42) Carter P, Kelley RF, Rodrigues ML, Snedecor B, Covarrubias M, Velligan MD, Wong WL, Rowland AM, Kotts CE, Carver ME, et al. High level Escherichia coli expression and production of a bivalent humanized antibody fragment. Biotechnology (N Y). 1992 Feb;10(2):163-7.
43) Venturi M, Seifert C, Hunte C. "High level production of functional antibody Fab fragments in an oxidizing bacterial cytoplasm." J. Mol. Biol. (2002) 315, 1-8.
44) Lindmark R. Fixed protein A-containing staphylococci as solid-phase immunoadsorbents. J Immunol Methods. 1982 Jul 30;52(2):195-203.
45) Guss B, Eliasson M, Olsson A, Uhlén M, Frej AK, Jörnvall H, Flock JI, Lindberg M. Structure of the IgG-binding regions of streptococcal protein G. EMBO J. 1986 Jul;5(7):1567-75.
46) Schmidt TG1, Koepke J, Frank R, Skerra A. Molecular interaction between the Strep-tag affinity peptide and its cognate target, streptavidin. J Mol Biol. 1996 Feb 9;255(5):753-66.
47) Thakur A, Lum LG. Cancer therapy with bispecific antibodies: Clinical experience. Curr Opin Mol Ther. 2010 Jun;12(3):340-9.
48) Burges A, Wimberger P, Kümper C, Gorbounova V, Sommer H, Schmalfeldt B, Pfisterer J, Lichinitser M, Makhson A, Moiseyenko V, Lahr A, Schulze E, Jäger M, Ströhlein MA, Heiss MM, Gottwald T, Lindhofer H, Kimmig R. Effective relief of malignant ascites in patients with advanced ovarian cancer by a trifunctional anti-EpCAM x anti-CD3 antibody: a phase I/II study. Clin Cancer Res. 2007 Jul 1;13(13):3899-905.
49) Kuwahara M, Kuroki M, Arakawa F, Senba T, Matsuoka Y, Hideshima T, Yamashita Y, Kanda H. A mouse/human-chimeric bispecific antibody reactive with human carcinoembryonic antigen-expressing cells and human T-lymphocytes. Anticancer Res. 1996 Sep-Oct;16(5A):2661-7.
50) Topp MS, Kufer P, Gökbuget N, Goebeler M, Klinger M, Neumann S, Horst HA, Raff T, Viardot A, Schmid M, Stelljes M, Schaich M, Degenhard E, Köhne-Volland R, Brüggemann M, Ottmann O, Pfeifer H, Burmeister T, Nagorsen D, Schmidt M, Lutterbuese R, Reinhardt C, Baeuerle PA, Kneba M, Einsele H, Riethmüller G, Hoelzer D, Zugmaier G, Bargou RC. Targeted therapy with the T cell-engaging antibody blinatumomab of chemotherapy-refractory minimal residual disease in B-lineage acute lymphoblastic leukemia patients results in high response rate and prolonged leukemia-free survival. J Clin Oncol. 2011 Jun 20;29(18):2493-8.
51) Cruz CR, Micklethwaite KP, Savoldo B, Ramos CA, Lam S, Ku S, Diouf O, Liu E, Barrett AJ, Ito S, Shpall EJ, Krance RA, Kamble RT, Carrum G, Hosing CM, Gee AP, Mei Z, Grilley BJ, Heslop HE, Rooney CM, Brenner MK, Bollard CM, Dotti G. Infusion of donor-derived CD19-redirected virus-specific T cells for B-cell malignancies relapsed after allogeneic stem cell transplant: a phase 1 study. Blood. 2013 Oct 24;122(17):2965-73.
52) Till BG, Jensen MC, Wang J, Qian X, Gopal AK, Maloney DG, Lindgren CG, Lin Y, Pagel JM, Budde LE, Raubitschek A, Forman SJ, Greenberg PD, Riddell SR, Press OW. CD20-specific adoptive immunotherapy for lymphoma using a chimeric antigen receptor with both CD28 and 4-1 BB domains: pilot clinical trial results. Blood. 2012 Apr 26;119(17):3940-50.
53) Ritchie DS, Neeson PJ, Khot A, Peinert S, Tai T, Tainton K, Chen K, Shin M, Wall DM, Hönemann D, Gambell P, Westerman DA, Haurat J, Westwood JA, Scott AM, Kravets L, Dickinson M, Trapani JA, Smyth MJ, Darcy PK, Kershaw MH, Prince HM. Persistence and efficacy of second generation CAR T cell against the LeY antigen in acute myeloid leukemia. Mol Ther. 2013 Nov;21(11):2122-9.
54) J.S. Patton et al. The lungs as a portal of entry for systemic drug delivery. Proc. Amer. Thoracic Soc. 2004 Vol. 1 pages 338-344.
55) Meidan VM, Michniak BB. Emerging technologies in transdermal therapeutics. Am J Ther. 2004 Jul-Aug;11(4):312-6.
56) Gennaro, A.L. and Gennaro, A.R. (2000) Remington: The Science and Practice of Pharmacy, 20th Ed., Lippincott Williams & Wilkins, Philadelphia, PA
57) Tan SM. The leucocyte β2 (CD18) integrins: the structure, functional regulation and signalling properties. Biosci Rep. 2012 Jun;32(3):241-69.
58) Edward F. Plow, Thomas A. Haas, Li Zhang, Joseph Loftus and Jeffrey W. Smith Ligand Binding to Integrins. July 21, 2000. The Journal of Biological Chemistry, 275, 21785-21788.
59) Lee DW, Gardner R, Porter DL, Louis CU, Ahmed N, Jensen M, Grupp SA, Mackall CL. Current concepts in the diagnosis and management of cytokine release syndrome. Blood. 2014 Jul 10;124(2):188-95.
60) Sanchez-Madrid F., Nagy J.A., Robbins E., Simon P., Springer T. (1983) The lymphocyte function-associated antigen (LFA-1), the C3bi complement receptor (OKM1/Mac-1), and the p150,95 molecule. J. Exp. Med. Vol. 158, p. 1785-1803
61) David V, Leca G, Corvaia N, Le Deist F, Boumsell L, Bensussan A. (1991) Proliferation of resting lymphocytes is induced by triggering T cells through an epitope common to the three CD18/CD11 leukocyte adhesion molecules. Cell Immunol. 136(2):519-24
62) Hildreth JE, Gotch FM, Hildreth PD, McMichael AJ. (1983) A human lymphocyte-associated antigen involved in cell-mediated lympholysis. Eur J Immunol. 13(3):202-8
63) Vermot Desroches C, Rigal D, Andréoni C. (1991) Regulation and functional involvement of distinct determinants of leucocyte function-associated antigen 1 (LFA-1) in T-cell activation in vitro. Scand J Immunol. 33(3):277-86
64) Ricevuti G, Mazzone A, Pasotti D, Fossati G, Mazzucchelli I, Notario A (1993) The role of integrins in granulocyte dysfunction in myelodysplastic syndrome. Leuk Res. 17(7):609-19
65) Beatty PG, Ledbetter JA, Martin PJ, Price TH, Hansen JA (1983) Definition of a common leukocyte cell-surface antigen (Lp95-150) associated with diverse cell-mediated immune functions. J Immunol. 131 (6):2913-8
66) Vedder N.B., Winn R.K., Rice C.L., Chi E.Y., Arfors K.E. Harlan J.M. (1990) Inhibition of leukocyte adherence by anti-CD18 monoclonal antibody attenuates reperfusion injury in the rabbit ear. Natl. Acad. Sci. Vol. 87, pp. 2643-2646
67) Jung et al. Int J Cancer Local immunotherapy of glioma patients with a combination of 2 bispecific antibody fragments and resting autologous lymphocytes: evidence for in situ t-cell activation and therapeutic efficacyJan 15;91(2):225-30, 2001

SEQUENCE LISTING

<110>  Deutsches Krebsforschungszentrum
       Eberhard Karls Universitaet Tuebingen

<120>  USE OF BLOCKING-REAGENTS FOR REDUCING UNSPECIFIC T
       CELL-ACTIVATION

<130>  DKF15102EP

<160>  5

<170>  PatentIn version 3.5

<210>  1
<211>  107
<212>  PRT
<213>  Artificial Sequence

<220>
<223>  CD3 single variable domain (clone UCHT1) VL

<400>  1

<210>  2
<211>  122
<212>  PRT
<213>  Artificial Sequence

<220>
<223>  CD3 single variable domain (clone UCHT1) VH

<400>  2

<210>  3
<211>  106
<212>  PRT
<213>  Artificial Sequence

<220>
<223>  CD3 single variable domain (clone OKT1) VL

<400>  3

Asp Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Leu Ala Ser Gly Val Pro Ala His Phe Arg Gly Ser
    50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn
            100                 105

<210>  4
<211>  119
<212>  PRT
<213>  Artificial Sequence

<220>
<223>  CD3 single variable domain (clone OKT1) VH

<400>  4

<210>  5
<211>  504
<212>  PRT
<213>  Artificial Sequence

<220>
<223>  Blinatumumab single chain variable fragment fusion protein (bite)

<400>  5

Asp Ile Gln Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp

20                  25                  30

Gly Asp Ser Tyr Leu Asn Trp Tyr Gln Gln Ile Pro Gly Gln Pro Pro
        35                  40                  45

Lys Leu Leu Ile Tyr Asp Ala Ser Asn Leu Val Ser Gly Ile Pro Pro
    50                  55                  60

Arg Phe Ser Gly Ser Gly Ser Gly Thr Asp Phe Thr Leu Asn Ile His
65                  70                  75                  80

Pro Val Glu Lys Val Asp Ala Ala Thr Tyr His Cys Gln Gln Ser Thr
                85                  90                  95

Glu Asp Pro Trp Thr Phe Gly Gly Gly Thr Lys Leu Glu Ile Lys Gly
            100                 105                 110

Gly Gly Gly Ser Gly Gly Gly Gly Ser Gly Gly Gly Gly Ser Gln Val
        115                 120                 125

Gln Leu Gln Gln Ser Gly Ala Glu Leu Val Arg Pro Gly Ser Ser Val
    130                 135                 140

Lys Ile Ser Cys Lys Ala Ser Gly Tyr Ala Phe Ser Ser Tyr Trp Met
145                 150                 155                 160

Asn Trp Val Lys Gln Arg Pro Gly Gln Gly Leu Glu Trp Ile Gly Gln
                165                 170                 175

Ile Trp Pro Gly Asp Gly Asp Thr Asn Tyr Asn Gly Lys Phe Lys Gly
            180                 185                 190

Lys Ala Thr Leu Thr Ala Asp Glu Ser Ser Ser Thr Ala Tyr Met Gln
        195                 200                 205

Leu Ser Ser Leu Ala Ser Glu Asp Ser Ala Val Tyr Phe Cys Ala Arg
    210                 215                 220

Arg Glu Thr Thr Thr Val Gly Arg Tyr Tyr Tyr Ala Met Asp Tyr Trp
225                 230                 235                 240

Gly Gln Gly Thr Thr Val Thr Val Ser Ser Gly Gly Gly Gly Ser Asp
                245                 250                 255

Ile Lys Leu Gln Gln Ser Gly Ala Glu Leu Ala Arg Pro Gly Ala Ser
            260                 265                 270

Val Lys Met Ser Cys Lys Thr Ser Gly Tyr Thr Phe Thr Arg Tyr Thr
        275                 280                 285

Met His Trp Val Lys Gln Arg Pro Gly Gln Gly Leu Glu Trp Ile Gly
    290                 295                 300

Tyr Ile Asn Pro Ser Arg Gly Tyr Thr Asn Tyr Asn Gln Lys Phe Lys
305                 310                 315                 320

Asp Lys Ala Thr Leu Thr Thr Asp Lys Ser Ser Ser Thr Ala Tyr Met
                325                 330                 335

Gln Leu Ser Ser Leu Thr Ser Glu Asp Ser Ala Val Tyr Tyr Cys Ala
            340                 345                 350

Arg Tyr Tyr Asp Asp His Tyr Cys Leu Asp Tyr Trp Gly Gln Gly Thr
        355                 360                 365

Thr Leu Thr Val Ser Ser Val Glu Gly Gly Ser Gly Gly Ser Gly Gly
    370                 375                 380

Ser Gly Gly Ser Gly Gly Val Asp Asp Ile Gln Leu Thr Gln Ser Pro
385                 390                 395                 400

Ala Ile Met Ser Ala Ser Pro Gly Glu Lys Val Thr Met Thr Cys Arg
                405                 410                 415

Ala Ser Ser Ser Val Ser Tyr Met Asn Trp Tyr Gln Gln Lys Ser Gly
            420                 425                 430

Thr Ser Pro Lys Arg Trp Ile Tyr Asp Thr Ser Lys Val Ala Ser Gly
        435                 440                 445

Val Pro Tyr Arg Phe Ser Gly Ser Gly Ser Gly Thr Ser Tyr Ser Leu
    450                 455                 460

Thr Ile Ser Ser Met Glu Ala Glu Asp Ala Ala Thr Tyr Tyr Cys Gln
465                 470                 475                 480

Gln Trp Ser Ser Asn Pro Leu Thr Phe Gly Ala Gly Thr Lys Leu Glu
                485                 490                 495

Leu Lys His His His His His His
            500

## Claims

1. Blocking-reagent for use in reducing unspecific T cell activation in therapy, the therapy comprising
administering to a subject a bispecific antibody molecule and/or a chimeric antigen receptor (CAR) modified T cell,
wherein the bispecific antibody molecule comprises two binding sites
i) wherein the first binding site binds to an antigen associated with a target cell and
ii) wherein the second binding site binds to a T cell receptor (TCR)/CD3 complex, on an effector cell and/or
wherein the CAR comprises
iii) an antibody molecule comprising a binding site that binds to an antigen associated with a target cell, and
iv) a TCR/CD3 signaling domain.

2. Blocking-reagent for use of claim 1, wherein the target cell expresses a tumor associated antigen (TAA) and/or an antigen associated with autoimmune diseases.

3. Blocking-reagent for use of claim 2, wherein the TAA is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1 R, (CD115), CD123, CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate blocking protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, Tie2, mesothelin, epithelial glycoprotein 2 (EGP2), epithelial glycoprotein 40 (EGP40), cancer antigen 72-4 (CA72-4), interleukin 13 receptor alpha-2 subunit, IL13Rα2, Ig kappa light chain (κ), GD3-ganglioside (GD3), GD2-ganglioside (GD2), CD171, NCAM, alpha folate receptor (αFR), Lewis(Y), fetal acetylcholine receptor (FAR), avian erythroblastic leukemia viral oncogene homolog 3 (ERBB3), avian erythroblastic leukemia viral oncogene homolog 4 (ERBB4), avian erythroblastic leukemia viral oncogene homolog 2 (ERBB2), hepatocyte growth factor receptor (HGFR/c-Met), claudin 18.2, claudin 3, claudin 4, claudin 1, claudin 12, claudin 2, claudin 5, claudin 8, claudin 7 and CD138.

4. Blocking-reagent for use of any of claims 1-3, wherein the blocking-reagent is selected from the group consisting of an antibody, a divalent antibody fragment, a monovalent antibody fragment, and a proteinaceous binding molecule with antibody-like binding properties.

5. Blocking-reagent for use of any of claims 1-4, wherein the blocking-reagent binds to a cell adhesion molecule or a cytokine.

6. Blocking-reagent for use of claim 5, wherein the cell adhesion molecule is selected the group consisting of CD18, CD11a, CD11b, CD11c, ICAM-1 (CD54), ICAM-2 (CD102), LFA1, LFA2 (CD2), CD58, CD86, CD80, OX-40 (CD134), 4-1 BB and/or LICOS (CD275) and/or the cytokine is selected from TNFalpha.

7. Blocking-reagent for use of any of claims 1-6, wherein the blocking-reagent reduces the activation of effector cells caused by a bystander cell.

8. Blocking-reagent for use of any of claims 1-7, wherein the first binding site of the bispecific antibody binds to a tumor associated antigen (TAA) as defined in claim 3 and/or wherein the CAR comprises an antibody molecule comprising a binding site that binds to a TAA as defined in claim 3.

9. Blocking-reagent for use of any of claims 1-8, wherein the blocking-reagent and the bispecific antibody molecule and/or the CAR modified T cell are administered simultaneously or sequentially.

10. Blocking-reagent for use of any of claims 1-9, wherein therapy is a therapy for treating a proliferatory disease or an autoimmune disease.

11. Blocking-reagent for use of any of claims 1-10, wherein side-effects of the therapy are reduced.

12. Blocking-reagent for use of any of claims 1-12, wherein in therapy the dosage of the administered bispecific antibody molecule and/or CAR modified T cell is increased compared to the dosage used without the blocking-reagent.

13. A pharmaceutical kit of parts, comprising in two separate parts:
a) a blocking-reagent, and
b) a bispecific antibody molecule,
wherein the bispecific antibody molecule binds to
i) a first antigen, and
ii) a T cell receptor (TCR)/CD3 complex.

14. A pharmaceutical kit of parts, comprising, in two separate parts:
a) a blocking-reagent, and
b) a chimeric antigen receptor (CAR) modified T cell
wherein the CAR comprises
i) an antibody molecule and
ii) a TCR/CD3 signaling domain.

15. An *in vitro* method for evaluating unspecific T cell activation,
the method comprising
(i) contacting bystander cells and effector cells with bispecific antibody molecules as defined in claim 1 that do not bind to the bystander cells, or
(ii) contacting bystander cells and effector cells with CAR T cells as defined in claim 1 that do not bind to the bystander cells, and
(iii) measuring unspecific T cell activation.
